# EUROPEAN PATENT SPECIFICATION

(11) **EP 2 340 303 B1**
(45) Date of publication and mention of the grant of the patent: **12.10.2016**
(21) Application number: 09822335.7
(22) Date of filing: 23.10.2009
(51) Int. Cl.: C12N 5/02

(54) **AMNIOTIC FLUID CELLS AND USES THEREOF**
AMNIONFLÜSSIGKEITSZELLEN UND VERWENDUNGEN DAFÜR
CELLULES DE LIQUIDE AMNIOTIQUE ET LEURS UTILISATIONS

(30) Priority: 24.10.2008 US 108313 P
(43) Date of publication of application: 06.07.2011
(73) Proprietor: CHILDRENS HOSPITAL LOS ANGELES, Los Angeles, CA 90027 (US)
(72) Inventor: PERIN, Laura, Los Angeles CA 90027 (US); DE FILIPPO, Roger, Glendale CA 91201 (US); WARBURTON, David, La Canada CA 91011 (US); DA SACCO, Stefano, Beverley Hills CA 90211 (US)
(74) Representative: Lucas, Brian Ronald
(86) International application number: PCT/US2009/005779
(87) International publication number: WO 2010/047824

(56) References cited:
- WO-A1-2005/075636
- US-A1- 2007 190 647
- US-A1- 2008 050 347
- US-A1- 2008 112 939
- US-A1- 2008 214 487
- US-A1- 2008 260 703
- L. Perin ET AL: "Renal differentiation of amniotic fluid stem cells", Cell Prolif., 1 January 2007 (2007-01-01), pages 936-948, XP55043675, Retrieved from the Internet: URL:http://onlinelibrary.wiley.com/store/1 0.1111/j.1365-2184.2007.00478.x/asset/j.13 65-2184.2007.00478.x.pdf?v=1&t=h9b67gp0&s= e79dc78af19ca6f2da143c120bf1ec63087eba5a [retrieved on 2012-11-09]
- PERIN LAURA ET AL: "Characterization of human amniotic fluid stem cells and their pluripotential capability", 1 January 2008 (2008-01-01), STEM CELL CULTURE; [METHODS IN CELL BIOLOGY], AMSTERDAM [U.A.] : ELSEVIER, ACAD. PRESS, NL, PAGE(S) 85 - 99, XP008133213, ISBN: 978-0-12-373876-9 * the whole document *
- PERIN LAURA ET AL: "Stem cell and regenerative science applications in the development of bioengineering of renal tissue", PEDIATRIC RESEARCH, WILLIAMS AND WILKINS, BALTIMORE, MD, US, vol. 63, no. 5, 1 May 2008 (2008-05-01), pages 467-471, XP009143011, ISSN: 0031-3998, DOI: 10.1203/PDR.0B013E3181660653
- CARRARO GIANNI ET AL: "Human amniotic fluid stem cells can integrate and differentiate into epithelial lung lineages", STEM CELLS, ALPHAMED PRESS, INC, UNITED STATES, vol. 26, no. 11, 1 January 2008 (2008-01-01), pages 2902-2911, XP009114182, ISSN: 1549-4918, DOI: 10.1634/STEMCELLS.2008-0090
- M. H. LITTLE: "Regrow or Repair: Potential Regenerative Therapies for the Kidney", JOURNAL OF THE AMERICAN SOCIETY OF NEPHROLOGY, vol. 17, no. 9, 9 August 2006 (2006-08-09) , pages 2390-2401, XP55047550, ISSN: 1046-6673, DOI: 10.1681/ASN.2006030218
- DA SACCO S ET AL: "Human Amniotic Fluid as a Potential New Source of Organ Specific Precursor Cells for Future Regenerative Medicine Applications", JOURNAL OF UROLOGY, LIPPINCOTT WILLIAMS & WILKINS, BALTIMORE, MD, US, vol. 183, no. 3, 1 March 2010 (2010-03-01) , pages 1193-1200, XP026938444, ISSN: 0022-5347, DOI: 10.1016/J.JURO.2009.11.006 [retrieved on 2010-03-01]
- LAURA PERIN ET AL: "Protective Effect of Human Amniotic Fluid Stem Cells in an Immunodeficient Mouse Model of Acute Tubular Necrosis", PLOS ONE, vol. 5, no. 2, 1 January 2010 (2010-01-01) , page e9357, XP055047750, ISSN: 1932-6203, DOI: 10.1371/journal.pone.0009357
- PETER V HAUSER ET AL: "Stem Cells Derived from Human Amniotic Fluid Contribute to Acute Kidney Injury Recovery", AMERICAN JOURNAL OF PATHOLOGY; [10640], AMERICAN SOCIETY FOR INVESTIGATIVE PATHOLOGY, US, vol. 177, no. 4, 1 October 2010 (2010-10-01), pages 2011-2021, XP002662436, ISSN: 0002-9440, DOI: 10.2353/AJPATH.2010.091245 [retrieved on 2010-11-29]
- HUMPHREYS ET AL: "The contribution of adult stem cells to renal repair", NEPHROLOGIE & THERAPEUTIQUE, ELSEVIER, NL, vol. 3, no. 1, 23 March 2007 (2007-03-23), pages 3-10, XP005936418, ISSN: 1769-7255
- CHALLEN G A ET AL: "Identifying the molecular phenotype of renal progenitor cells", JOURNAL OF THE AMERICAN SOCIETY OF NEPHROLOGY, WILLIAMS AND WILKINS, BALTIMORE, MD, US, vol. 15, no. 9, 1 September 2004 (2004-09-01), pages 2344-2357, XP002454239, ISSN: 1046-6673, DOI: 10.1097/01.ASN.0000136779.17837.8F

## Description

### Field of the Invention

This invention relates to the field of stem cells and progenitor cells obtained from amniotic fluid and methods of isolation, culture, differentiation and use thereof.

### Background of the Invention

Acute and chronic renal diseases are a major health issue all over the world. The number of patients with end-stage renal disease (ESRD) is increasing in both developed and developing countries (Thadhani et al., 1996). As reported by the U.S. Renal Data System Annual Data Report, in 2005, more than 400,000 Americans were affected by ESRD and more than 20,000 were waiting for a kidney transplant. According to predictions for 2020 it is expected that more than 500,000 Americans will be affected by ESRD.

Amniotic fluid has been used as a safe and reliable screening tool for genetic and congenital diseases in the fetus for many years. The volume and composition of the amniotic fluid changes with the physiological variations of the developing fetus during pregnancy. The molecular composition of amniotic fluid and the presence of nutritive substances have been shown to have a key role in the proliferation and differentiation of various intestinal cell types such as epithelial and mucosa cells (Cellini et al., 2006).

Contact between amniotic fluid and compartments of the developing fetus, such as lung and gastrointestinal tract, may explain the presence of different cell types in the milieu of amniotic fluid. Mature cell lines derived from all three germ layers including mesenchymal, hematopoietic cells and cells expressing proteins and various markers from specific tissue types such as brain, heart and pancreas have all been discovered in amniotic fluid (Hoehn and Salk, 1982; Gosden, 1983; Torricelli et al., 1993). However, further investigation is required to completely categorize these cells according to their origin and function (Tsangaris et al., 2004; Bossolasco et al., 2006; McLaughlin et al., 2006).

### Summary of the Invention

Applicants have characterized the amniotic fluid total cell population focusing on cells from the three germ layers and on progenitor cells for organs by following their presence over time and investigating the variations in cellular amniotic composition occurring during pregnancy. A subpopulation of cells presenting characteristics of kidney progenitors including tubular and glomeruli precursors has been isolated from amniotic fluid.

The invention provides amniotic fluid derived renal progenitor cells positive for CD24, OB-cadherin and podocalixin, being an isolated and purified population or a clonal population or progeny differentiated from said cells. In an embodiment, the cells have the capacity to be induced to differentiate to metanephric, podocyte, glomerular, distal and proximal epithelial tubular, stromogenic mesenchymal or mesanglial cells. In an embodiment, the population of cells can be induced to differentiate *in vivo* or *ex vivo.*

One embodiment provides a composition comprising a population of amniotic fluid derived renal progenitor cells as defined above and a pharmaceutically acceptable carrier and/or culture medium. Another embodiment provides a method to prepare a composition comprising mixing the amniotic fluid derived renal progenitor cells as defined above or progeny differentiated therefrom and a carrier (e.g., cell culture medium).

Another embodiment provides a method of producing a population of amniotic fluid derived renal progenitor cells comprising selecting CD24, OB-cadherin and podocalixin positive cells from an amniotic fluid sample. In one embodiment, the selecting is performed using an antibody against CD24, an antibody against OB-cadherin and an antibody against podocalixin (e.g., monoclonal antibody, polyclonal antibody, or an antibody conjugated to a fluorochrome or a magnetic particle). In one embodiment, the selecting is performed by flow cytometry such as fluorescence activated cell sorting or high gradient magnetic selection.

One embodiment provides for an isolated and purified population of amniotic fluid derived renal progenitor cells as defined above prepared by any of the methods described herein.

Another embodiment provides a method to proliferate a population of cells enriched for amniotic fluid derived renal progenitor cells comprising: selecting at least one CD24, OB-cadherin and podocalixin positive cell from an amniotic fluid sample; introducing said at least one cell to a culture medium; and proliferating said at least one selected cell in the culture medium.

One embodiment provides a method to differentiate an isolated and purified population of amniotic fluid derived renal progenitor cells positive for CD24, OB-cadherin and podocalixin comprising contacting said population with at least one differentiation factor, including, but not limited to, ATRA, Vitamin 3, Dexamethasone, BMP-7, many different types of collagen IV or a combination thereof.

Another embodiment provides a method of storing an isolated and purified population of amniotic fluid derived renal progenitor cells positive for CD24, OB-cadherin and podocalixin comprising obtaining an amniotic fluid sample from a human subject; isolating a population of CD24, OB-cadherin and podocalixin positive renal progenitor cells from said sample; and cryopreserving the amniotic fluid derived renal progenitor cells positive for CD24, OB-cadherin and podocalixin.

There is also disclosed a method to prevent or treat kidney disease or injury comprising administering to a subject (e.g., a mammal including a human) an amount of amniotic fluid derived renal progenitor cells positive for CD24, OB-cadherin and podocalixin or progeny differentiated therefrom effective to treat the disease or injury. Also disclosed is a method to prevent or treat kidney disease or injury comprising administering to a subject an amount of a population of c-kit positive cells isolated and purified from amniotic fluid or progeny differentiated therefrom effective to treat the disease or injury.

The cells may be administered by local or systemic injection. The disease may comprise diabetic nephropathy, membranous nephropathy, focal segmental glomerulosclerosis, membranoproliferative glomerulonephritis, diffuse proliferative glomerulonephritis, membranous focal segmental glomerulosclerosis, mild glomerular lesions, mesangial proliferative glomerulonephritis, intraductal proliferative glomerulonephritis, mesangial capillary glomerulonephritis, high-density precipitation glomerulonephritis, crescentic glomerulonephritis, sclerosing glomerulonephritis, ischemic nephropathy, glomerular disease based on systematic diseas, glomerular diseases based on vascular disease, glomerular disease based on metabolic diseases, hereditary renal lesions or transplanted glomerular lesions. The disease may be Alport's syndrome. The injury may be a result of physical trauma (e.g., due to surgery or an accident or chemical (e.g., chemical overdose)).

The invention also provides amniotic fluid derived renal progenitor cells as defined above for use in treating kidney damage as a result of an injury or disease.

One embodiment provides for the use of an isolated and purified population of amniotic fluid derived renal progenitor cells as defined above produced by any of the methods disclosed herein to prepare a medicament for treating kidney damage as a result of an injury or disease. In one embodiment, the medicament includes a physiologically acceptable carrier and/or cell culture medium.

### Brief Description of the Figures

Figure 1 depicts the panel of markers used for the characterization of human amniotic fluid populations for the three germ layers and organ progenitor cells by RT-PCR and Western blotting analysis.
Figure 2 depicts the panel of markers used for the characterization of human amniotic fluid populations for pluripotent cells by RT-PCR and Western blotting analysis.
Figure 3 depicts the panel of markers used for the characterization of human amniotic fluid populations and derived subpopulations for kidney commitment by RT-PCR and Western blotting analysis.
Figure 4 depicts the expression of markers for the three germ layers over time by RT-PCR (A) and Western blotting (B).
Figure 5 depicts RT-PCR of CD24⁺OB-Cadherin⁺ population compared with a CD24⁻OB Cadherin⁻ selection.
Figure 6 depicts expression of markers for kidney progenitor cells in samples of different gestational age by RT-PCR (A) and Western blotting (B).
Figure 7 depicts RT-PCR of CD24⁺OB-Cadherin⁺ derived subpopulations.
Figure 8 depicts morphology of total amniotic fluid cell population (A, B) and of CD24⁺OB-Cadherin⁺ cell population (C, D).
Figure 9 depicts expression of markers for progenitor cells in samples of different gestational age by RT-PCR (A) and Western Blotting (B).

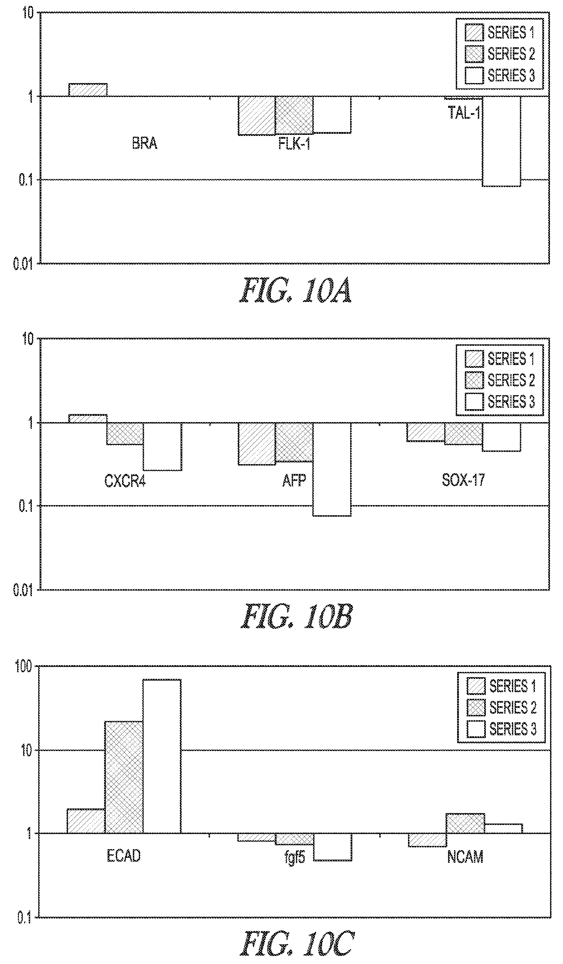

Figure 10 depicts Real Time PCR showing expression of markers for mesoderm, endoderm and ectoderm.

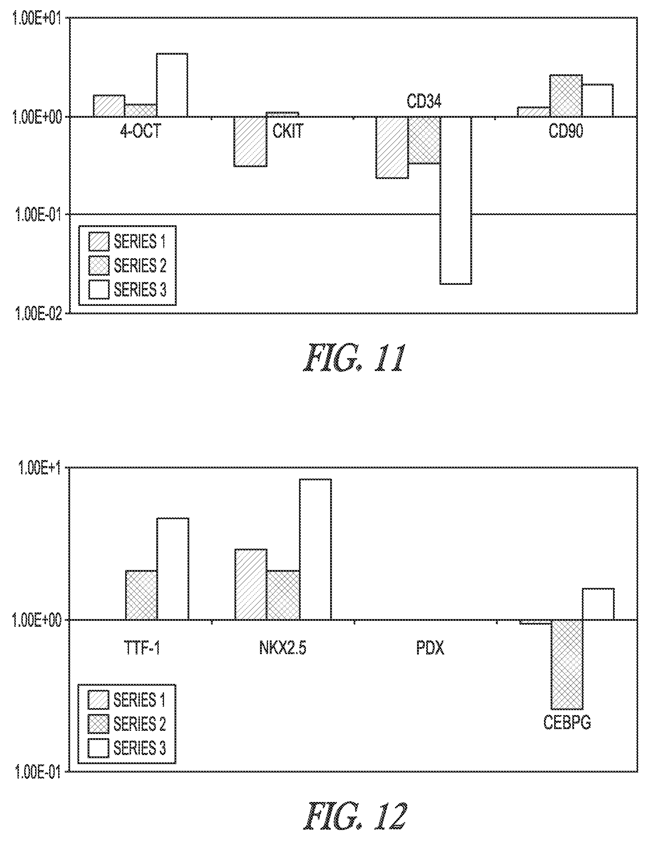

Figure 11 depicts Real Time PCR for pluripotency, hematopoietic and mesenchymal markers.
Figure 12 depicts Real Time PCR for progenitor markers.

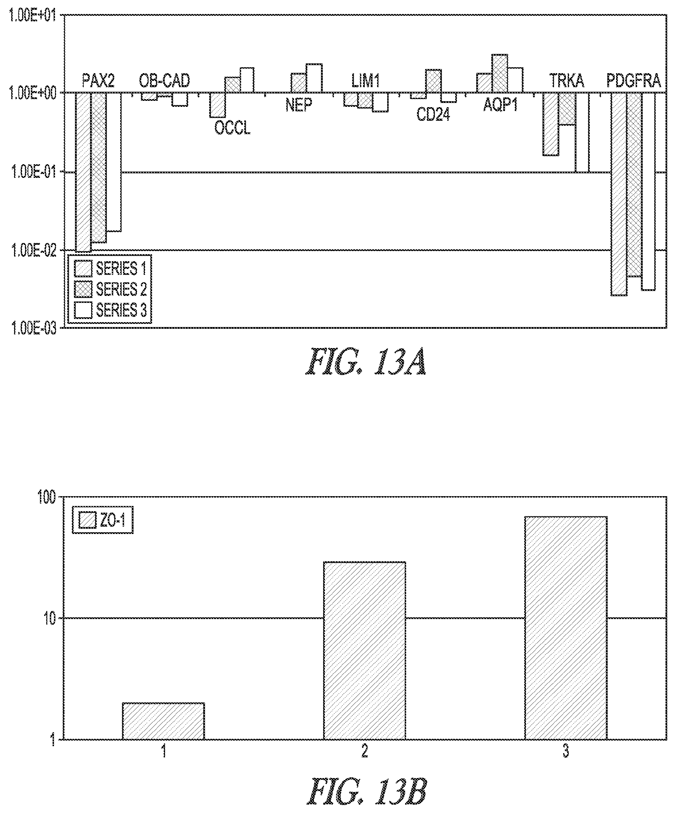

Figure 13 depicts Real Time PCR for kidney progenitors.
Figure 14 depicts a list of the human primers, the size of the products and the annealing temperature used in the experiments.
Figures 15A-C: A. Morphology of hAFSC population. After 40 passages in culture under bright field (10x) the cells present a fibroblastoid appearance. B. RT-PCR of hAFSC before the injection. No early and mature kidney markers are expressed. *β*-actin is used as housekeeping gene (390bp). C. Karyotype of hAFSC after 38 passages. The cells do not present any abnormality and have a normal karyotype.

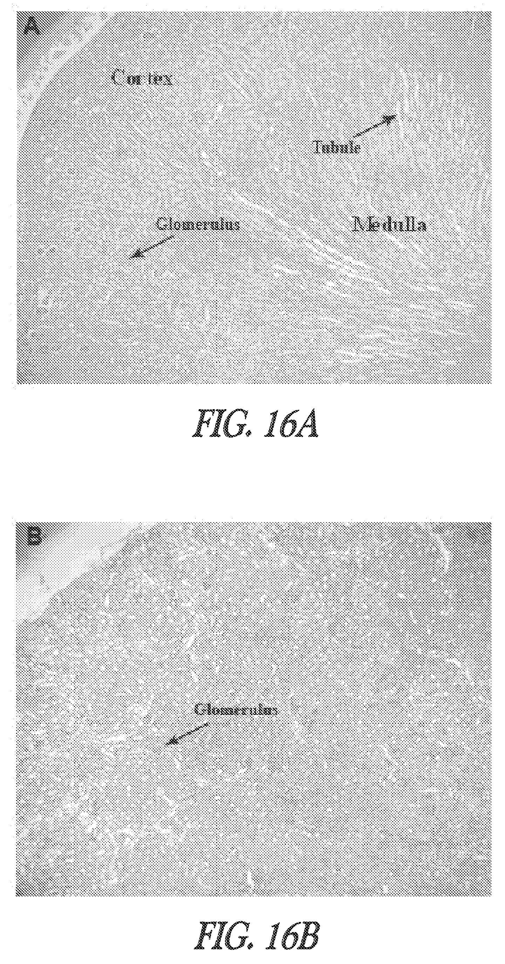

Figures 16A-D: A. Histological section (H&E) of a nu/nu mouse kidney. Cortex is readily distinguishable from the medulla. Tubules and glumeruli, indicated by the arrow, present normal morphology (10x). B. Histological section (H&E) of a nu/nu mouse kidney after 3 days of glycerol-rhabodomyolysis-induced ATN. The glomeruli are still present (arrow) and not damaged, while the tubules are damaged (10x). C. Histological section (PAS Staining) of a nu/nu mouse kidney. The tubules and the glomerulai are intact and they present normal morphology when compared with the injured ones. (D) Where it is noticeable, intraluminar cast formation (arrow), destruction of the brush borders (arrow) and disorganization of the structures.

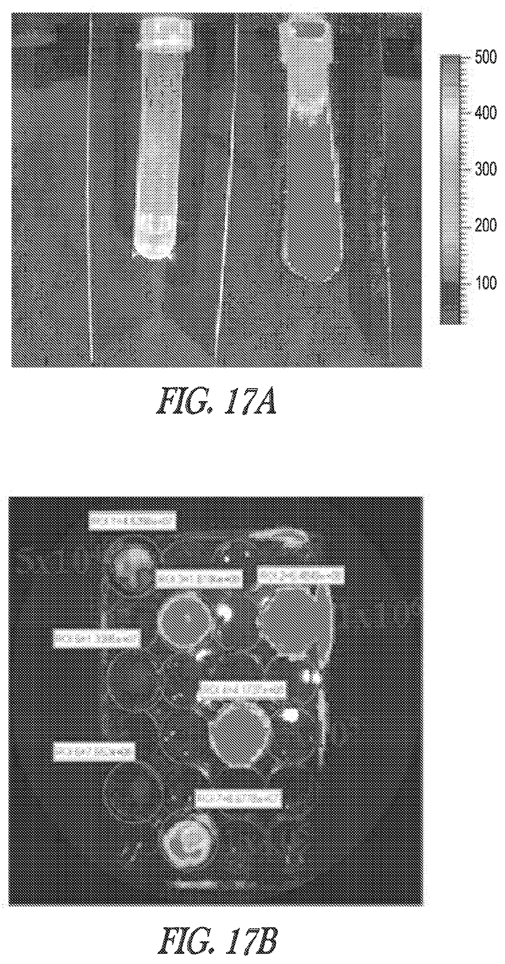

Figures 17A-D: A. The control (non-infected hAFSC on the left) and the transduced hAFSC with lentivirus coding for luciferase (on the right) were exposed to the substrate for the luciferase (luciferin) and it was determined by luminescence that luciferase expression in AFSC persists after *20pds in vitro.* B. *In vitro* experiment to determine the smallest amount of AFSC that are detected under bioluminescence imaging. It was established that 1x10⁵ cells was the minimum number of cells that could be injected in order to detect a signal. C. *In vivo* experiment of bioluminescent detection of AFSC after injection in a damaged nu/nu mouse kidney. 1.2x10⁶ AFSC were injected directly into the right kidney and the left kidney was used as a control. In panel 1 demonstrates the expression of luciferase right after the injection. The signal is very strong at 5hr (panel 2) and also after 24 hr (panel 3). The signal starts to decrease around day 6 (panel 5) and it is not evident on the following days (panel 6-7). It appears again after 20 days as shown in panel 8. D. RT-PCR demonstrated the presence of the luciferase sequence (500bp) in injected kidneys, compared with the cells before injection (positive control) and in un-injected kidney (negative control). *β*-actin was used as housekeeping gene (390bp). E. Immuno-fluorescence staining of injected kidney with hAFSC after 3 weeks. The red fluorescence (arrow) confirms the presence of hAFSC expressing luciferase. The nuclei are stained with dapi (20x).

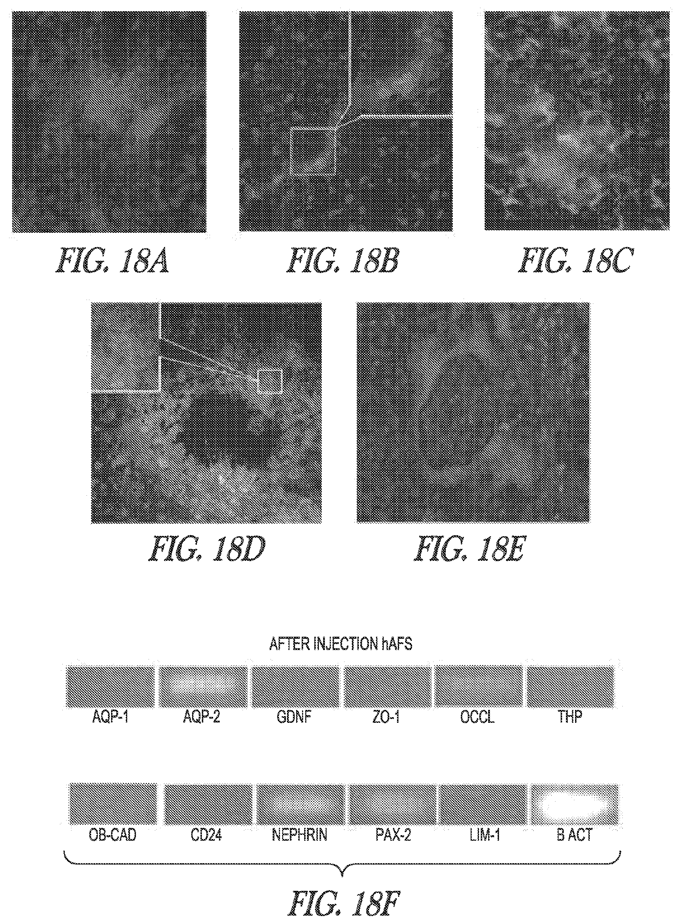

Figures 18A-F: A. Frozen section of an injected kidney with hAFSC after 1 week. The cells are evident under red fluorescence given by the surface marker CM-Dil. The nuclei are stained with DAPI (30x). B. Double Immuno-florescence staining of injected kidney with hAFSC after 3 weeks. Positive expression for Aqp2 is shown by the red florescence while green florescence indicates expression of luciferase. Double staining on the same cells expressing both of the markers in a tubule (arrow). The nuclei are stained with DAPI (30x). C. Immuno-fluorescence staining of injected kidney with hAFSC after 3 weeks. The red fluorescence indicates hAFSC labeled with the surface marker CM-DiI. It is noticeable that hAFSC locate in proximity of the tubular structure. The green fluorescence indicates the expression of Peanut Agglutinin by hAFSC. The nuclei are stained with DAPI (40x). D. Immuno-fluorescence staining of injected kidney with hAFSC after 3 weeks. The red fluorescence indicates hAFSC labeled with the surface marker CM-Dil. It is noticeable that hAFSC locate in proximity of the tubular structure. The green fluorescence indicates the expression of Dolichus Biflorus Agglutinin by hAFSC (arrow). The nuclei are stained with dapi (40x). E. Immuno-fluorescence staining of injected kidney with hAFSC after 3 weeks. The red fluorescence indicates hAFSC labeled with the surface marker CM-Dil. It is noticeable that hAFSC locate in close proximity of the glumerulai structure. The green fluorescence indicates the expression of Glial Derived Neurotrophic Factor by hAFSC (arrow). The nuclei are stained with DAPI (40x). F. RT-PCR of injected hAFSC after 3 weeks. The cells expressed NPHS1, AQP2, PAX2, OCLN, ACTB is used as a housekeeping gene.

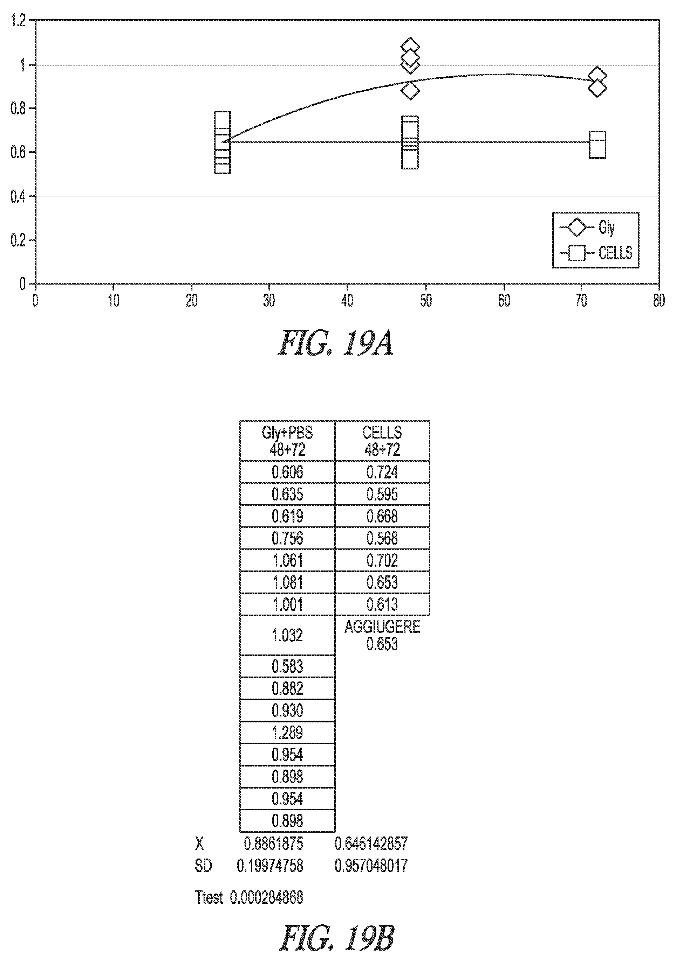

Figure 19 depicts a graph demonstrating the effect of hAFSC on the level of blood creatinine when injected into nu/nu mice that underwent glycerol-induced ATN, over a period of 3 days. The control group (only damage and damage plus vehicle control, PBS) show an increase in the level of creatinine between 48h and 72h, while the mice treated with the hAFSC did not show a peak and they maintain the level of creatinine close to physiological parameters, 0.6mg/dl.

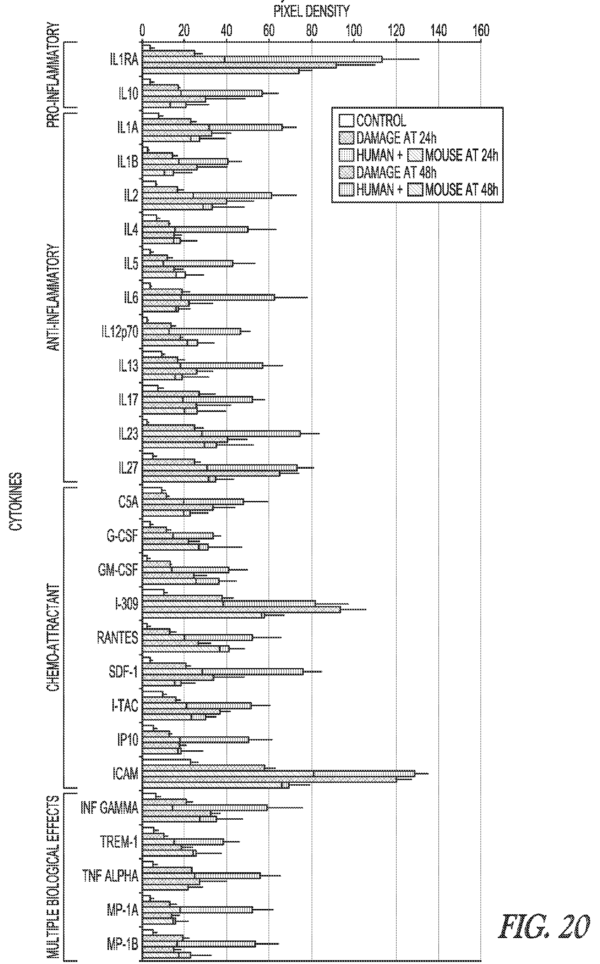

Figure 20 provides a suite of bar graphs detailing the profile of mouse and human cytokines that are expressed in mouse kidney in control mice (green), in mice with glycerol-induced-rhabdomyolysis ATN (blue) versus mice with injection into the kidney of hAFSC simultaneous with the intramuscular glycerol injection to induce ATN (mouse derived cytokines shown as red hatched bars, human cytokines as red bars). Values are mean ± SD. The cytokines were divided into 4 broad functional groups based on their principal biological activity during inflammation: 1. Anti-inflammatory; 2. Pro-inflammatory; 3. Chemoattractants; and 4. Multiple biological affects. The profile of cytokines expressed in whole mouse kidney was evaluated at 24h and at 48h.
Figure 21 depicts a summary of the genes expressed in sub-populations AKPC as measured by Real-Time PCR.
Figure 22 depicts the different cell lineagyes identified within amniotic fluid (AF).

### Detailed Description of the Invention

Applicants have characterized the amniotic fluid total cell population focusing on cells from the three germ layers and on progenitor cells for organs by following their presence over time and investigating the variations in cellular amniotic composition occurring during pregnancy. A subpopulation of cells presenting characteristics of kidney progenitors including tubular and glomeruli precursors has been isolated from amniotic fluid. The cells have use in, for example, medicinal therapy and research.

### Definitions

As used herein, the terms below are defined by the following meanings:
"Progenitor cells" are cells produced during differentiation of a stem cell that have some, but not all, of the characteristics of their terminally-differentiated progeny. Defined progenitor cells, such as "renal progenitor cells," are committed to a lineage (renal), but not to a specific or terminally-differentiated cell type.

The terms "isolated" or an "enriched population" refers to a cell or cells which are not associated with one or more cells or one or more cellular components that are associated with the cell or cells *in vivo.*

A "subject" is a vertebrate, such as a mammal, including a human. Mammals include, but are not limited to, humans, farm animals, sport animals and companion animals. Included in the term "animal" is dog, cat, fish, gerbil, guinea pig, hamster, horse, rabbit, swine, mouse, monkey (e.g., ape, gorilla, chimpanzee, orangutan) rat, sheep, goat, cow and bird. Subjects that can benefit from the cells and methods of the invention include, but are not limited to, those suffering from a loss of function of kidney cells as a result of physical or disease related damage.

An "effective amount" generally means an amount which provides the desired local or systemic effect and/or performance, particularly for treating a condition of interest. For example, an effective dose is an amount sufficient to affect a beneficial or desired clinical result. Said dose could be administered in one or more administrations and could include any preselected amount of cells. The precise determination of what would be considered an effective dose may be based on factors individual to each subject, including their size, age, size of the damage, and amount of time since the damage occurred or the disease began. One skilled in the art, specifically a physician, would be able to determine the number of cells that would constitute an effective dose.

"Expansion" refers to the propagation of cells without differentiation.

"Progressive kidney disease" as used herein refers to any disease of the kidney that over time (e.g., days, weeks, months, years) leads to a loss of renal function.

"Renal function" generally refers to a physiological property of the kidney, such as the ability to retain protein thereby preventing proteinuria (e.g., urinary creatinine, the excretion of protein in an amount greater than about 0.15 g/24 hours). Renal function can be assessed, for example, by glomerular filtration rate (e.g., creatinine clearance), excretion of protein in urine, blood urea nitrogen, serum or plasma creatinine, or any combination thereof.

"Podocytes" are specialized, highly differentiated pericyte-like cells of the visceral epithelium in the kidneys. Podocytes form a crucial component of the glomerular filtration barrier, contributing size and charge selectivity and maintaining a massive filtration surface. "Pedicels" (or "foot processes") extend from the podocyte. These delicate foot processes cover the exterior basement membrane surface of the glomerular capillary. Adjacent podocytes interdigitate to cover the basal lamina which is intimately associated with the glomerular capillaries; however, gaps or thin filtration slits remain. When podocytes contract, they cause closure of the filtration slits. This decreases the glomerular filtration rate (GFR) by reducing the surface area available for filtration. Furthermore, podocytes are known to synthesize matrix molecules to the glomerular basement membrane (GBM), including type IV collagen, laminin, entactin, and agrin. Podocyte injury or loss leads to proteinuria, where large amounts of protein are lost from the blood. Moreover, loss of podocytes is a hallmark of diabetic and nondiabetic progressive chronic kidney disease (CKD).

"Self-renewal" refers to the ability to produce replicate daughter cells having differentiation potential that is identical to those from which they arose. A similar term used in this context is "proliferation."

As used herein, "treat," "treating" or "treatment" includes treating, reversing, preventing, ameliorating, or inhibiting an injury or disease-related condition or a symptom of an injury or disease-related condition.

"Co-administer" can include simultaneous and/or sequential administration of two or more agents/cell types.

The terms "comprises," "comprising," and the like can have the meaning ascribed to them in U.S. Patent Law and can mean "includes," "including" and the like. As used herein, "including" or "includes" or the like means including, without limitation.

### Isolation, Growth and Characterization of Amniotic Fluid-Derived Renal Progenitor Cells

The present invention relates to an isolated and purified population of amniotic fluid derived renal progenitor cells positive for CD24 and OB-cadherin and methods of isolation, culture, differentiation and use thereof. The isolation, growth and characterization of which are discussed in detail in the Examples below.

Additionally, during and after isolation, the amniotic fluid derived renal progenitor cells of the invention can be cultured in culture medium that is well established in the art and commercially available from the American Type Culture Collection (ATCC). Such media include, but are not limited to, Dulbecco's Modified Eagle's Medium (DMEM), DMEM F12 medium, Eagle's Minimum Essential Medium, F-12K medium, Iscove's Modified Dulbecco's Medium, or RPMI-1640 medium. It is within the skill of one in the art to modify or modulate concentrations of media and/or media supplements as needed for the cells used. It will also be apparent that many media are available as low-glucose formulations, with or without sodium pyruvate.

Also contemplated is supplementation of cell culture medium with mammalian sera. Sera often contain cellular factors and components that are necessary for viability and expansion. Examples of sera include fetal bovine serum (FBS), bovine serum (BS), calf serum (CS), fetal calf serum (FCS), newborn calf serum (NCS), goat serum (GS), horse serum (HS), human serum, chicken serum, porcine serum, sheep serum, rabbit serum, rat serum (RS), serum replacements, and bovine embryonic fluid. It is understood that sera can be heat-inactivated at 55-65°C if deemed necessary to inactivate components of the complement cascade. Modulation of serum concentrations, or withdrawal of serum from the culture medium can also be used to promote survival of one or more desired cell types. In one embodiment, the amniotic fluid derived renal progenitor cells are cultured in the presence of FBS /or serum specific for the species cell type. For example, amniotic fluid derived renal progenitor cells can be isolated and/or expanded with total serum (e.g., FBS) concentrations of about 0.5% to about 5% or greater including about 5% to about 15%. Concentrations of serum can be determined empirically.

Additional supplements can also be used to supply the cells with trace elements for optimal growth and expansion. Such supplements include insulin, transferrin, sodium selenium, and combinations thereof. These components can be included in a salt solution such as, but not limited to, Hanks' Balanced Salt Solution® (HBSS), Earle's Salt Solution®, antioxidant supplements, MCDB-201® supplements, phosphate buffered saline (PBS), N-2-hydroxyethylpiperazine-N'-ethanesulfonic acid (HEPES), nicotinamide, ascorbic acid and/or ascorbic acid-2-phosphate, as well as additional amino acids. Many cell culture media already contain amino acids; however some require supplementation prior to culturing cells. Such amino acids include, but are not limited to, L-alanine, L-arginine, L-aspartic acid, L-asparagine, L-cysteine, L-cystine, L-glutamic acid, L-glutamine, L-glycine, L-histidine, L-inositol, L-isoleucine, L-leucine, L-lysine, L-methionine, L-phenylalanine, L-proline, L-serine, L-threonine, L-tryptophan, L-tyrosine, and L-valine.

Antibiotics are also typically used in cell culture to mitigate bacterial, mycoplasmal, and fungal contamination. Typically, antibiotics or anti-mycotic compounds used are mixtures of penicillin/streptomycin, but can also include, but are not limited to, amphotericin (Fungizone®), ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Hormones can also be advantageously used in cell culture and include, but are not limited to, D-aldosterone, diethylstilbestrol (DES), dexamethasone, β-estradiol, hydrocortisone, insulin, prolactin, progesterone, somatostatin/human growth hormone (HGH), thyrotropin, thyroxine, and L-thyronine. β-mercaptoethanol can also be supplemented in cell culture media.

Lipids and lipid carriers can also be used to supplement cell culture media, depending on the type of cell and the fate of the differentiated cell. Such lipids and carriers can include, but are not limited to cyclodextrin (α, βγ), cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulation.

Cells in culture can be maintained either in suspension or attached to a solid support, such as extracellular matrix components and synthetic or biopolymers. Cells often require additional factors that encourage their attachment to a solid support (e.g., attachment factors) such as type I, type II, and type IV collagen, concanavalin A, chondroitin sulfate, fibronectin, "superfibronectin" and/or fibronectin-like polymers, gelatin, laminin, poly-D and poly-L-lysine, Matrigel™, thrombospondin, and/or vitronectin.

The maintenance conditions of cells can also contain cellular factors that allow cells, such as the amniotic fluid derived renal progenitor cells of the invention, to remain in an undifferentiated form. It may be advantageous under conditions where the cell must remain in an undifferentiated state of self-renewal for the medium to contain epidermal growth factor (EGF), platelet derived growth factor (PDGF), leukemia inhibitory factor (LIF), basic fibroblast growth factor (bFGF) and combinations thereof. It is apparent to those skilled in the art that supplements that allow the cell to self-renew (e.g., to produce replicate daughter cells having differentiation potential that is identical to those from which they arose; a similar term used in this context is "proliferation"), but not differentiate should be removed from the culture medium prior to differentiation. It is also apparent that not all cells will require these factors. In fact, these factors may elicit unwanted effects, depending on the cell type.

Amniotic fluid derived renal progenitor cells of the invention can be selected based on the markers (gene and/or protein) described herein. Accordingly, positive selection methods can be used, either alone or together with the methods described above, to identify and/or isolate the cells of the invention. Methods of positive selection can include visual selection, using microscopy and/or other means of detection, including, but not limited to, immunoblotting, immunofluorescence, and/or enzyme-linked immunosorbent assay. Other methods of positive selection can also include, but are not limited to, additional selective culture techniques (e.g., variable cell densities or amounts of CO₂), flow cytometry, RT-PCR, and/or microchip-based methods of cell separation. Negative selection methods may also be used.

### Inducing Amniotic Fluid Derived Renal Progenitor Cells to Differentiate

Using appropriate growth factors, chemokines and/or cytokines, amniotic fluid derived renal progenitor cells of the invention can be induced to differentiate to form a number of cells. From the primary CD24⁺OB-Cadherin⁺ selection five different subpopulations of progenitors were isolated from the different cell lineages derived from metanephric mesenchyme.

Podocyte progenitors were identified with two populations expressing podocalyxin and nephrin, respectively. To direct the cells to a more complete differentiation ATRA (1-10 microM), Vitamin D3 (100 nM) and/or Dexamethasone (100 nM for the first 24-48 hours) in different combinations were used to culture the cells, seeded onto collagen IV coated wells.

Mesangial progenitor cells were identified by the expression of PDGF Receptor Alpha and were differentiated by adding to the culture media PDGF Alpha and/or Beta.

An E-Cadherin⁺ subpopulation was selected for progenitors of the glomerular epithelial cells, leading to maturation using BMP-7 while Stromogenic Mesenchymal cells, expressing TrKA, were differentiated adding VEGF to the culture media.

### Uses for Amniotic Fluid-Derived Renal Progenitor Cells

Amniotic fluid derived renal progenitor cells of the invention can be used for the generation of kidney lineages, including but not limited to, podocytes, glomerular cell types, distal and proximal epithelial tubular cells and mesangial cells.

Therefore, one embodiment provides methods for providing kidney cells, which can include, but are not limited to, podocytes, glomerular cell types, distal and proximal epithelial tubular cells and mesangial cells, comprising differentiating amniotic fluid derived renal progenitor cells of the invention in the presence of differentiation factors and isolating the cells. The differentiation factors can be, but are not limited to, ATRA, Vitamin 3, Dexamethasone, BMP-7 and/or many different types of collagen IV or combination thereof. Differentiation can occur *in vitro, in vivo* or *ex vivo.*

Amniotic fluid derived renal progenitor cells of the invention and other fastidious cells can benefit from co-culturing with another cell type. Such co-culturing methods arise from the observation that certain cells can supply yet-unidentified cellular factors that allow the cell to differentiate into a specific lineage or cell type. These cellular factors can also induce expression of cell-surface receptors, some of which can be readily identified by monoclonal antibodies. Generally, cells for co-culturing can be selected based on the type of lineage one skilled in the art wishes to induce, and it is within the abilities of the skilled artisan to select the appropriate cells for co-culture.

Methods of identifying and subsequently isolating differentiated cells from their undifferentiated counterparts can be carried out by methods well known in the art. Cells that have been induced to differentiate can be identified by selectively culturing cells under conditions whereby differentiated cells outnumber undifferentiated cells. These conditions include, for example, extending the amount of time that cells are grown in culture, such that survival of a desired cell type is encouraged. Many primary cells achieve senescence, and fail to divide, or die, after a period of time. Other conditions comprise modulating the type and concentration of serum, or culturing the cells in the presence or absence of growth factors and/or cytokines that induce differentiation to another cell type. Differentiation can also be advantageously achieved by modulation of serum concentrations, or withdrawal of serum from the culture. Other methods of inducing differentiation can include, but are not limited to, modulating the acidity of the culture medium, as well as the oxygen and carbon dioxide levels during culture.

Similarly, differentiated cells can be identified by morphological changes and characteristics that are not present on their undifferentiated counterparts, such as cell size, the number of cellular processes, and the complexity of intracellular organelle distribution. Also contemplated are methods of identifying differentiated cells by their expression of specific cell-surface markers such as cellular receptors and transmembrane proteins. Monoclonal antibodies against these cell-surface markers can be used to identify differentiated cells. Detection of these cells can be achieved through fluorescence activated cell sorting (FACS), and/or enzyme-linked immunosorbent assay (ELISA). From the standpoint of transcriptional upregulation of specific genes, differentiated cells often display levels of gene expression that are different from undifferentiated cells. Reverse-transcription polymerase chain reaction (RT-PCR) can also be used to monitor changes in gene expression in response to differentiation. In addition, whole genome analysis using microarray technology can be used to identify differentiated cells.

Accordingly, once differentiated cells are identified, they can be separated from their undifferentiated counterparts, if necessary. The methods of identification detailed above also provide methods of separation, such as FACS, preferential cell culture methods, ELISA, magnetic beads, and combinations thereof. One embodiment of the invention envisions the use of FACS to identify and separate cells based on cell-surface antigen expression. It is understood that the methods of identification and separation are not limited to analysis of differentiated cell types, but can also be used to identify undifferentiated cell types such as the amniotic fluid derived renal progenitor cells of the invention.

Amniotic fluid derived renal progenitor cells of the invention can also be used in cell replacement therapies. Amniotic fluid derived renal progenitor cells of the invention can be administered to a tissue of interest in a subject to supplement functioning cells or replace cells, which have lost function. Alternatively, methods of providing differentiated cells are also contemplated, wherein the amniotic fluid derived renal progenitor cells of the invention are differentiated in the presence of differentiation factors, isolated, and administered into or upon the body of a subject. In one embodiment, the differentiated cells are cells of the renal lineage.

Disease states characterized by loss of kidney mass and/or function, and that could benefit from amniotic fluid derived renal progenitor cells and methods of the invention include, but are not limited to, kidney disease or injury. For example, one therapeutic use of the cells of the invention is for treating a subject with a renal disease, promoting growth of new tissue in a subject, or promoting survival of damaged tissue in a subject. Therapeutic use of the cells of the invention includes treatment of kidney diseases. The kidney disease can be, for example, a result or a consequence of any change, damage, or trauma to the glomerulus, tubules or interstitial tissue in either the renal cortex or renal medulla of the kidney. The kidney disease can be acute or chronic.

A subject may be regarded as being in, or at risk of, chronic renal failure, or at risk of needing renal replacement therapy, if that subject has already been diagnosed as afflicted with, or would be regarded as being afflicted with, a condition which typically leads to progressive loss of renal function associated with progressive loss of functioning nephron units. Such conditions or causes include, but are not limited to, chronic renal failure, end-stage renal disease, hypertensive nephrosclerosis, hereditary nephritis, renal dysplasia, diabetes, sepsis, dehydration, medication (e.g., excessive water loss due to diuretic intake, NSAIDs including ibuprofen and naproxen, antibiotics like aminoglycosides (gentamicin, tobramycin,) lithium, and iodine-containing mediacations), loss of blood supply due to obstruction of the renal artery or vein, rhabdomyolysis, multiple myeloma, systemic lupus erthematosus, obstruction of bladder, high blood pressure, prostatic hypertrophy or prostate cancer, renal cancer, tumors, kidney stones and the like.

In some embodiments, the kidney disease is a progressive kidney disease. In some embodiments, the kidney disease is a progressive glomerular kidney disease. Progressive glomerular kidney diseases that are particularly suitable for treatment by the methods described herein include, for example, diabetic nephropathy (e.g., as a consequence of Type I or Type II diabetes or systemic lupus), primary glomerulonephritis (e.g., membranous nephropathy, focal segmental glomerulosclerosis, membranoproliferative glomerulonephritis, diffuse proliferative glomerulonephritis, membranous focal segmental glomerulosclerosis, mild glomerular lesions, mesangial proliferative glomerulonephritis including IgA nephropathy, intraductal proliferative glomerulonephritis, mesangial capillary glomerulonephritis, high-density precipitation glomerulonephritis, crescentic glomerulonephritis including extratubal glomerulonephritis, and sclerosing glomerulonephritis) and secondary glomerulonephritis (e.g., ischemic nephropathy, glomerular disease based on systematic disease including lupus nephritis and Goodpasture syndrome, glomerular diseases based on vascular disease including glomerular thrombosis, glomerular disease based on metabolic diseases including diabetic nephropathy and amyloidosis, hereditary renal lesions including Alport's syndrome (which derives from a mutation of the alpha3, alpha4, or alpha5 chains of type IV collagen, that constitute basement membrane in the kidney, ear and eye), and transplanted glomerular lesions). Further kidney diseases include Finnish-type nephrosis, ischemia-reperfusion injury, congenital nephrotic syndrome, pyelonephritis, interstitial nephritis, acute tubular necrosis, pharmaceutical drug or toxicant renal disorder, acute nephritis syndrome, rapidly progressive glomerulonephritis syndrome, relapsing and continuous hematuria, and chronic nephritis syndrome.

Alport syndrome (AS) is a well-defined model for chronic kidney disease (CKD) leading to end-stage renal disease (ESRD). AS is a rare hereditary glomerulonephritis that affects 1 in 20,000 people. It is caused by genetic defects in type IV collagen, which leads to failure to produce a normal glumerular basement membrane (GBM). Kidney injury and eventual ESRD are associated with glomerular sclerosis and tubulointerstatial fibrosis, associated with fibroblast activation, inflammation and reorganization of extracellular matrix. Chronic nephritis progresses more rapidly to ESRD in males. In some families, AS may be associated with hearing loss and other disorders including the eue, skin, platelets, white blood cells, as well as smooth muscle tumors. Abut 80% of affected families show X-linked inheritance, while the remainder are autosomal dominant or recessive. At present, there is no definitive therapy to delay progression to ESRD for patients with Alport Syndrome. Two mouse models are available (129 col4alpha3-/- autosomal recessive and C57BL col4alpha5-/- X-linked) to study AS.

Amniotic fluid derived renal progenitor cells of the invention can be used for many diverse clinical and pre-clinical applications, which can include, but are not limited to, use in toxicological or genomic screening methods, determination of levels of enzymes, as well as treatment of the diseases disclosed herein. Amniotic fluid derived renal progenitor cells of the invention can provide a variety of differentiated cultured cell types for high-throughput toxicological or genomic screening. The cells can be cultured in, for example, 96-well or other multi-well culture plates to provide a system for high-throughput screening of, for example, target cytokines, chemokines, growth factors, or pharmaceutical compositions in pharmacogenomics or pharmacogenetics.

Thus, the present invention provides for use of amniotic fluid derived renal progenitor cells of the invention to detect cellular responses (e.g., toxicity) to bioactive (biologic or pharmacologic) agents, comprising contacting a culture of cells, or the differentiated progeny thereof, with one or more biologic or pharmacologic agents, identifying one or more cellular response to the one or more biologic or pharmacologic agents, and comparing the cellular responses of the cell cultures to the cellular responses of control cultures.

The invention also envisions a tissue-engineered organ, or portion, or specific section thereof, a tissue engineered device comprising a tissue of interest and optionally, cytokines, growth factors, or differentiation factors that induce differentiation into a desired cell type, wherein the amniotic fluid derived renal progenitor cells of the invention are used to generate kidney tissue. Tissue-engineered organs can be used with a biocompatible scaffold to support cell growth in a three-dimensional configuration, which can be biodegradable. Tissue-engineered organs generated from the amniotic fluid derived renal progenitor cells of the invention can be implanted into a subject in need of a replacement organ, portion, or specific section thereof. The present invention also envisions the use of the amniotic fluid derived renal progenitor cells of the invention or cells differentiated therefrom as part of a bioreactor.

Homogenous organs, portions, or sections derived from the amniotic fluid derived renal progenitor cells of the invention can be implanted into a host. Likewise, heterogeneous organs, portions, or sections derived from amniotic fluid derived renal progenitor cells of the invention induced to differentiate into multiple tissue types can be implanted into a subject in need thereof. The transplantation can be autologous, such that the donor of the cells from which organ or organ units are derived is the recipient of the engineered tissue. The transplantation can be heterologous, such that the donor of the cells from which organ or organ units are derived is not that of the recipient of the engineered-tissue (e.g., allogeneic or xenogenic).

Once transferred into a host, the tissue-engineered organs can recapitulate the function and architecture of the native host tissue. The tissue-engineered organs will benefit subjects in a wide variety of applications, including the treatment of cancer and other diseases disclosed herein, congenital defects, or damage due to surgical resection.

### Administration of Amniotic Fluid Derived Renal Progenitor Cells

For the purposes described herein, either autologous, allogeneic or xenogeneic amniotic fluid derived renal progenitor cells of the invention can be administered to a subject, either in differentiated or undifferentiated form, genetically altered or unaltered, by direct injection to a tissue site, systemically, on or around the surface of an acceptable matrix, encapsulated or in combination with a pharmaceutically acceptable carrier.

Amniotic fluid derived renal progenitor cells of the invention can be administered to a subject by a variety of methods known in the art. Amniotic fluid derived renal progenitor cells of the invention can be administered to a subject by localized or systemic injection.

In one embodiment, a cell suspension is drawn up into a syringe and administered to a subject. Multiple injections may be made using this procedure. The use of such cellular suspension procedures provides many advantages. For example, these methods direct cells to any predetermined site and are relatively non-traumatic.

Typically, the number of cells transplanted into a subject will be a "therapeutically effective amount." As used herein, a "therapeutically effective amount" refers to the number of transplanted cells that are required to effect treatment of the particular injury, or disease for which treatment is sought. For example, where the treatment is for tissue injury, transplantation of a therapeutically effective amount of cells will typically produce a reduction in the amount and/or severity of the symptoms associated with the injury. Persons of skill in the art will understand how to determine proper cell dosages.

As desired, amniotic fluid derived renal progenitor cells of the invention and their differentiated progeny can be induced to proliferate and/or differentiate *in vivo* by administering to the host, any growth factor(s), cytokine(s) or pharmaceutical composition(s) that will induce proliferation and differentiation of the cells. These growth factor(s), cytokine(s) or pharmaceutical composition(s) include any growth factor, cytokine or pharmaceutical composition known in the art, including the growth factors and cytokines described herein for *in vitro* proliferation and differentiation.

Exogenous factors (e.g., cytokines, differentiation factors and other factors) can be administered prior to, after or concomitantly with the amniotic fluid derived renal progenitor cells of the invention. For example, a form of concomitant administration would comprise combining a factor of interest in the culture media and/or pharmaceutically acceptable carrier prior to administration. Doses for administrations are variable, may include an initial administration followed by subsequent administrations; but nonetheless, can be ascertained by the skilled artisan, from this disclosure, the documents cited herein, and the knowledge in the art.

A parameter involved in the therapeutic use of amniotic fluid derived renal progenitor cells of the invention is the quantity of cells necessary to achieve an optimal effect. Different scenarios may require optimization of the amount of cells injected into a tissue of interest. For example, the quantity of cells to be administered will vary for the subject being treated. In one embodiment, between 10⁴ to 10⁸, more preferably 10⁵ to 10⁷, and most preferably 3 x 10⁷ cells and optionally, 50 to 500 µg/kg per day of a cytokine can be administered to a human subject. However, the precise determination of what would be considered an effective dose may be based on factors individual to each patient, including their size, age, size tissue damage, and amount of time since the damage occurred. Therefore, dosages can be readily ascertained by those skilled in the art from this disclosure and the knowledge in the art.

Another parameter involved in the use of amniotic fluid derived renal progenitor cells of the invention is the purity of the population. Amniotic fluid, for example, comprise mixed populations of cells, which can be purified to a degree sufficient to produce a desired effect. Those skilled in the art can readily determine the percentage of amniotic fluid derived renal progenitor cells of the invention in a population using various well-known methods, such as fluorescence activated cell sorting (FACS). Preferable ranges of purity in populations comprising amniotic fluid derived renal progenitor cells of the invention are about 1 to about 5%, about 5 to about 10%, about 10 to about 15%, about 15 to about 20%, about 20 to about 25%, about 25 to about 30%, about 30 to about 35%, about 35 to about 40%, about 40 to about 45%, about 45 to about 50%, about 50 to about 55%, about 55 to about 60%, about 60 to about 65%, about 65 to about 70%, about 70 to about 75%, about 75 to about 80%, about 80 to about 85%, about 85 to about 90%, about 90% to about 95% or about 95 to about 100%. Purity of the cells can be determined according to the cell surface marker profile within a population. Dosages can be readily adjusted by those skilled in the art (e.g., a decrease in purity may require an increase in dosage).

When administering a therapeutic composition of the present invention, it will generally be formulated in a unit dosage injectable form (solution, suspension, emulsion). The pharmaceutical formulations suitable for injection include sterile aqueous solutions and dispersions. The carrier can be a solvent or dispersing medium containing, for example, water, saline, phosphate buffered saline, polyol (for example, glycerol, propylene glycol, liquid polyethylene glycol, and the like) and suitable mixtures thereof.

Additionally, various additives which enhance the stability, sterility, and isotonicity of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. In many cases, it will be desirable to include isotonic agents, for example, sugars, sodium chloride, and the like. Prolonged absorption of the injectable pharmaceutical form can be brought about by the use of agents delaying absorption, for example, aluminum monostearate and gelatin. According to the present invention, however, any vehicle, diluent, or additive used would have to be compatible with the cells.

Sterile injectable solutions can be prepared by incorporating the cells utilized in practicing the present invention in the required amount of the appropriate solvent with various amounts of the other ingredients, as desired. Examples of compositions comprising progenitor cells of the invention include liquid preparations for administration, including suspensions; and, preparations for intramuscular or intravenous administration (e.g., injectable administration), such as sterile suspensions or emulsions. Such compositions may be in admixture with a suitable carrier, diluent, or excipient such as sterile water, physiological saline, glucose, dextrose, or the like. The compositions can also be lyophilized. The compositions can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "REMINGTON'S PHARMACEUTICAL SCIENCE", 17th edition, 1985, which is incorporated herein by reference, may be consulted to prepare suitable preparations, without undue experimentation.

Compositions of the invention are conveniently provided as liquid preparations, e.g., isotonic aqueous solutions, suspensions, emulsions or viscous compositions, which may be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues.

The choice of suitable carriers and other additives will depend on the exact route of administration and the nature of the particular dosage form, e.g., liquid dosage form (e.g., whether the composition is to be formulated into a solution, a suspension, gel or another liquid form, such as a time release form or liquid-filled form).

Solutions, suspensions and gels normally contain a major amount of water (e.g., purified, sterilized water) in addition to the cells. Minor amounts of other ingredients such as pH adjusters (e.g., a base such as NaOH), emulsifiers or dispersing agents, buffering agents, preservatives, wetting agents and jelling agents (e.g., methylcellulose), may also be present. The compositions can be isotonic, i.e., they can have the same osmotic pressure as blood and lacrimal fluid.

The desired isotonicity of the compositions of this invention may be accomplished using sodium chloride, or other pharmaceutically acceptable agents such as dextrose, boric acid, sodium tartrate, propylene glycol or other inorganic or organic solutes.

Viscosity of the compositions, if desired, can be maintained at the selected level using a pharmaceutically acceptable thickening agent. Methylcellulose is readily and economically available and is easy to work with. Other suitable thickening agents include, for example, xanthan gum, carboxymethyl cellulose, hydroxypropyl cellulose, carbomer, and the like. The concentration of the thickener will depend upon the agent selected. The point is to use an amount, which will achieve the selected viscosity. Viscous compositions are normally prepared from solutions by the addition of such thickening agents.

A pharmaceutically acceptable preservative or cell stabilizer can be employed to increase the life of the compositions. If preservatives are used, it is well within the purview of the skilled artisan to select compositions that will not affect the viability or efficacy of the amniotic fluid derived renal progenitor cells as described herein.

Those skilled in the art will recognize that the components of the compositions should be selected to be chemically inert. This will present no problem to those skilled in chemical and pharmaceutical principles, or problems can be readily avoided by reference to standard texts or by simple experiments (not involving undue experimentation), from this disclosure and the documents cited herein.

Compositions can be administered in dosages and by techniques well known to those skilled in the medical and veterinary arts taking into consideration such factors as the age, sex, weight, and condition of the particular patient, and the composition form used for administration (e.g., solid vs. liquid). Dosages for humans or other mammals can be determined without undue experimentation by the skilled artisan, from this disclosure, the documents cited herein, and the knowledge in the art.

Suitable regimes for initial administration and further doses or for sequential administrations also are variable and may include an initial administration followed by subsequent administrations; but nonetheless, can be ascertained by the skilled artisan, from this disclosure, the documents cited herein, and the knowledge in the art.

### Genetically-Modified Amniotic Fluid Derived Renal Progenitor Cells of the Invention

Amniotic fluid derived renal progenitor cells of the invention or differentiated progeny derived therefrom can be genetically altered. Amniotic fluid derived renal progenitor cells described herein or their differentiated progeny can be genetically modified by introducing heterologous DNA or RNA into the cell by a variety of recombinant methods known to those of skill in the art. These methods are generally grouped into four major categories: (1) viral transfer, including the use of DNA or RNA viral vectors, such as retroviruses, including lentiviruses, Simian virus 40 (SV40), adenovirus, alpha virus, including Sindbis virus, and bovine papillomavirus, for example; (2) chemical transfer, including calcium phosphate transfection and DEAE dextran transfection methods; (3) membrane fusion transfer, using DNA-loaded membranous vesicles such as liposomes, red blood cell ghosts and protoplasts, for example; and (4) physical transfer techniques, such as microinjection, microprojectile, electroporation, nucleofection or direct "naked" DNA transfer.

Cells can be genetically altered by insertion of pre-selected isolated DNA, by substitution of a segment of the cellular genome with pre-selected isolated DNA, or by deletion of or inactivation of at least a portion of the cellular genome of the cell. Deletion or inactivation of at least a portion of the cellular genome can be accomplished by a variety of means, including but not limited to genetic recombination, by antisense technology (which can include the use of peptide nucleic acids or PNAs), or by ribozyme technology, for example. Insertion of one or more pre-selected DNA sequences can be accomplished by homologous recombination or by viral integration into the host cell genome. Methods of non-homologous recombination are also known, for example, as described in U.S. Patent Nos. 6,623,958, 6,602,686, 6,541,221, 6,524,824, 6,524,818, 6,410,266, 6,361,972.

The desired gene sequence can also be incorporated into the cell, particularly into its nucleus, using a plasmid expression vector and a nuclear localization sequence. Methods for directing polynucleotides to the nucleus have been described in the art. For example, signal peptides can be attached to plasmid DNA to direct the DNA to the nucleus for more efficient expression.

The genetic material can be introduced using promoters that will allow for the gene of interest to be positively or negatively induced using certain chemicals/drugs, to be eliminated following administration of a given drug/chemical, or can be tagged to allow induction by chemicals (including but not limited to the tamoxifen responsive mutated estrogen receptor) in specific cell compartments (including, but not limited to, the cell membrane).

Any of the transfection or transduction techniques can also be applied to introduce a transcriptional regulatory sequence into amniotic fluid derived renal progenitor cells of the invention or progeny to activate a desired endogenous gene. This can be done by both homologous (e.g., U.S. 5,641,670) or non-homologous (e.g., U.S. 6,602,686) recombination.

Successful transfection or transduction of target cells can be demonstrated using genetic markers. The green fluorescent protein of *Aequorea victoria,* for example, has been shown to be an effective marker for identifying and tracking genetically modified hematopoietic cells. Alternative selectable markers include the β-Gal gene, the truncated nerve growth factor receptor, and drug selectable markers (including but not limited to NEO, MTX, hygromycin).

### Examples

The following examples are provided in order to demonstrate and further illustrate certain embodiments and aspects of the present invention and are not to be construed as limiting the scope thereof.

### Example 1

### Characterization of progenitors derived from human amniotic fluid

### Materials and Methods

### Expansion of human amniotic fluid total cell population

28 samples were obtained from amniocentesis from 15 to 20 weeks of gestational age. Samples with normal male karyotype and normal ultrasound were collected from discarded plates from Genzyme (Pasadena, CA). Cells were expanded in tissue culture dishes (BD Falcon, Franklin Lakes, NJ) with three different culture media: 1. Chang's media (alpha MEM, 20% Chang B and 2% Chang C) (Irvine Scientific, Santa Ana, CA), L-Glutamine 20% of ES-FBS (Gibco/Invitrogen, Carlsbad, CA) and 1% of antibiotic (Gibco/Invitrogen, Carlsbad, CA); 2. Amniomax II was added as supplied (Gibco); 3. DMEM was added with 10% FBS (Gibco/Invitrogen, Carlsbad, CA) and 1% antibiotic (Gibco/Invitrogen, Carlsbad, CA). Cells were trypsinized using trypsin 0.25% EDTA (Gibco/Invitrogen, Carlsbad, CA). Cells were cultured in an incubator at 37° Celsius and 5% CO₂ for 50 passages.

### Characterization of amniotic fluid by expression of markers for the three germ layers and progenitor cells

28 human AF samples were stratified by gestational agent and than assigned to be assayed using either RT-PCR or Real-Time PCR.

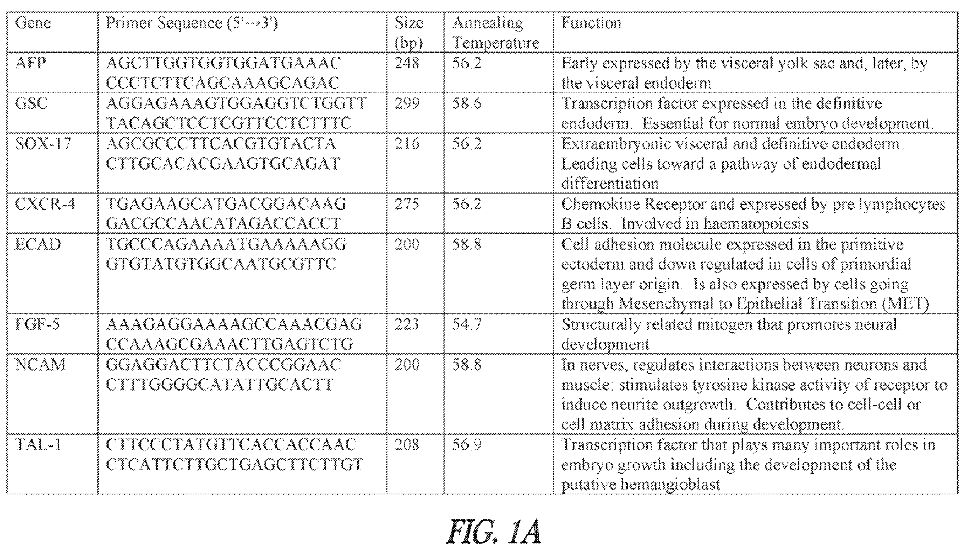

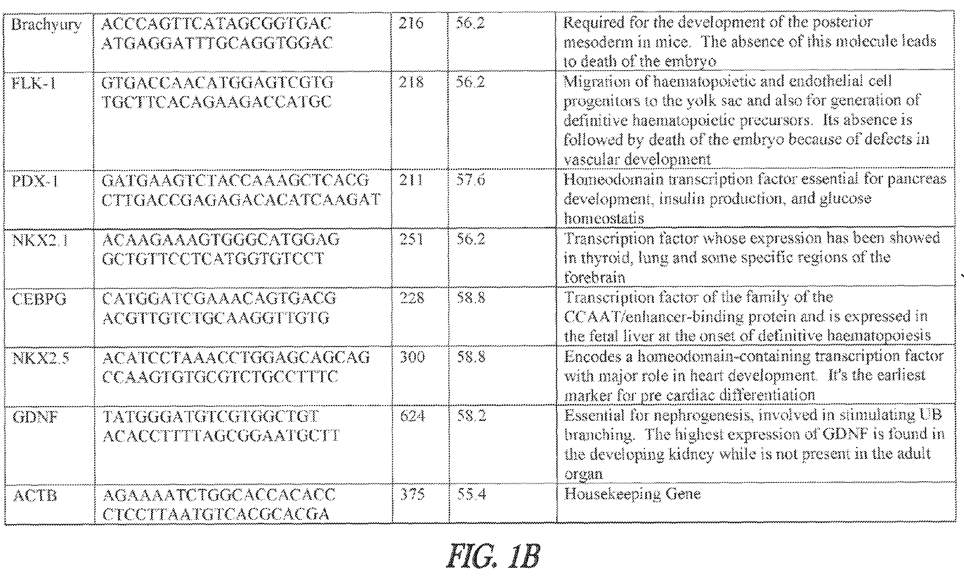

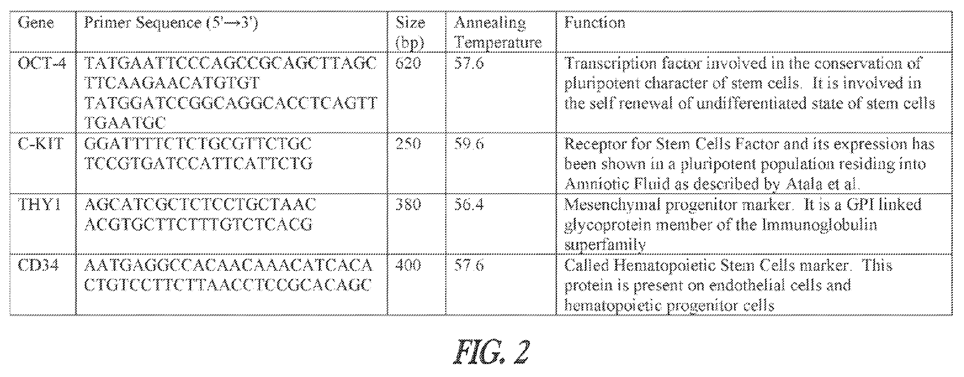

Sixteen samples of Amniotic Fluid were analyzed with RT-PCR and Western blotting for a wide panel of markers from all the three germ layers, mesenchymal and hematopoietic precursors and early progenitor cells from different organs (Figures 1, 2 and 3).

Twelve samples of AF were analyzed by real-time PCR to determine the quantitative variation in the expression of the different markers.

### Analysis and characterization by RT-PCR

Between passages 4 and 5 and after trypsinization, cells were collected for RT-PCR and Western blotting analysis. Total RNA was isolated using the RNeasy Mini Kit (Invitrogen,Carlsbad, CA) as described on the data sheet. Briefly, with the use of silica-gel columns RNA is separated from DNA through spin processes after lysis and homogenization of the samples. Ethanol addition allows RNA to bind the silica-gel before the spinning step. The RNA solution obtained was then processed with DNAse treatment (DNAse I, Invitrogen, Carlsbad, CA) to avoid any possible genomic contamination. One microgram of total RNA was reverse transcribed using SuperScript II reverse transcriptase (Invitrogen, Carlsbad, CA). The cDNA was amplified with Taq Polymerase (Invitrogen, Carlsbad, CA) in the presence of gene specific primers (Operon, Huntsville, AL). Amplification conditions were as follows: 94°C, 3 minutes; 94° C, 45 seconds; annealing temperature specific for each primer between 55° and 62° C, 30 seconds; 72°C, 1:30 minutes in a total of 36 cycles followed by 10 minutes at 72°C. RT-PCR products were separated in a 1.0% agarose/ethidium bromide gel and visualized using Blue/Orange Loading Dye (Promega, San Luis Obispo, CA).

Twelve samples of AF cells were analyzed by Real-Time PCT to quantitate the expression of specific markers previously mentioned. Real-Time PCT was carried out using a Roche Light Cycler 480 and Light Cycler TagMan MasterMix. 35 cycles were performed for each experiment with standard Real-Time PCR conditions.

### Analysis and characterization by Western blotting

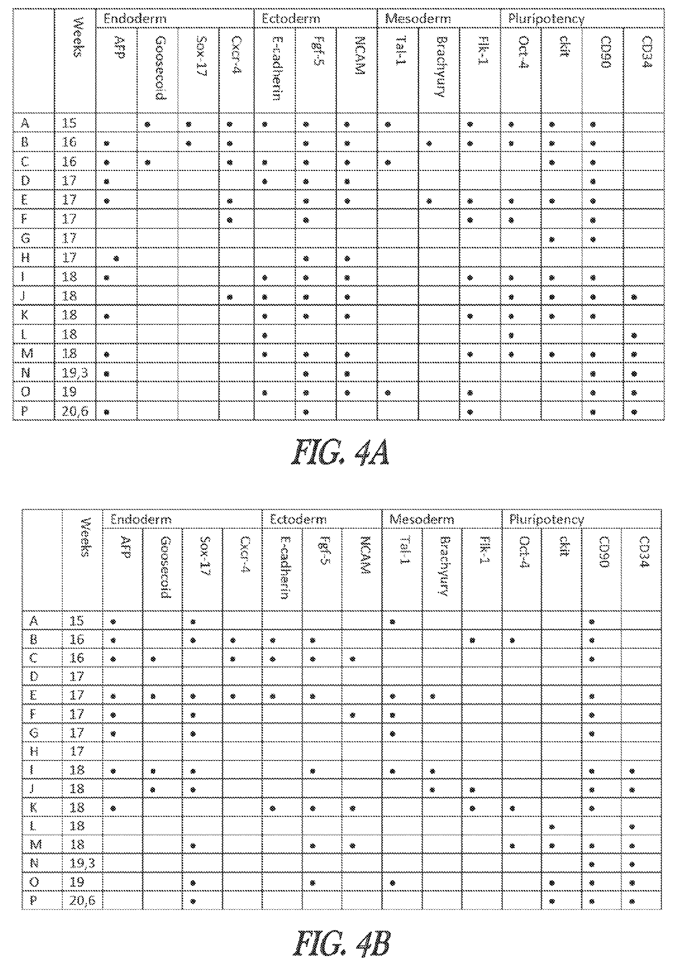

Total cell lysates were prepared using the Nuclear Extract Kit (Active Motif) following the manufacturer's instructions. After washing the plate with a phosphatase inhibitor, solution cells were scraped from the plate, collected, and centrifuged at 500 rpm for 5 minutes at 4° C. After incubation in lysis buffer at 4° C for 20 minutes cells were vortexed and successively centrifuged at 14000 rpm, for 20 minutes at 4° C. Supernatant was collected and concentration was measured with UV-VIS Spectroscopy. Each sample was prepared with Loading Buffer containing 250 mM Tris HCl (Sigma-Aldrich, St. Louis, MO) pH 6.8, 10% SDS (USBio, Cleveland, OH), 30% Glycerol (Sigma-Aldrich, St. Louis, MO), 5% B-Mercaptoethanol (Sigma-Aldrich, St. Louis, MO), 0.02% Bromophenol blue (Sigma-Aldrich, St. Louis, MO-Aldrich). Equal amounts (20 ml, 1ng/ul) of individual protein solution were separated from each sample, after a 1 minute boiling step, by SDS-PAGE with 4%-20% Glycin gels. Individual solutions were then transferred to PVDF 0.45 um membrane (Millipore, Billerica, MA) and probed with a various range of antibodies at 1:1000 concentration (Figure 4). Peroxide conjugation of secondary antibodies was performed (Sigma-Aldrich, St. Louis, MO) with concentrations as follows: 1:10000 for Anti-Mouse, 1:15000 for Anti-Rabbit, 1:8000 for anti-Chicken and 1:120000 for anti-Goat secondary antibodies. The blocking steps were performed with 10% Dry Fat Milk (Santa Cruz Biotech., Santa Cruz, CA) in TBS. TBS-T (1% Triton) was used as washing solution. Detection of antigens was performed using ECL Western Blotting detection Reagents (Amersham Biosciences/GE Healthcare, Buckinghamshire, UK), and impressed on Biomax Light Film (GE Healthcare, Buckinghamshire, UK) with a 1 minute exposure.

### Analysis and characterization by Real Time PCR

Total RNA was isolated and reverse transcribed as previously stated starting from an RNA concentration equal to 800ng/microliter. Quantitative real-time PCR was carried out using the Roche Light Cycler 480 and the Light Cycler TaqMan Master Mix.
Real Time PCR conditions were as follows: 90°C for 10 minutes, 60°C for 10 seconds, 72°C for 1 second with the analysis of the fluorescent emission at 72°C. 35 cycles were performed for each experiment. All primers and probes were generated by Roche.

### Selection and characterization of a renal progenitor population from whole amniotic fluid

### Immunoseparation of renal populations and subpopulations from whole amniotic fluid

A positive population for both CD24 and OB-Cadherin was selected by incubating the total amniotic fluid cells with specific antibodies (mouse monoclonal OB Cadherin: ABCAM AB52891 and mouse monoclonal CD24 ABCAM AB31622-100 diluted 1:100) for 30 minutes at 4° C on a rocking platform, followed by incubation with immunomagnetic microbeads for 5 minutes at 25° C and then 15 minutes at 4° C followed by immunoseparation by MS columns (Miltenyi Biotech, Germany). Positive and negative (used as a negative control) selected populations were replated on tissue culture dishes with Chang's Media for subsequent expansion.

A further immunoselection to identify four subpopulations of renal progenitors was performed as above described, using anti-Human antibodies for Nephrin, TrKa, PDGFR Alpha, podocalyxin and E-Cadherin following the previously described immunoseparation technique. Final subpopulations were obtained after a total of 18 passages from the original samples. Cells were reseeded under the same conditions used for the total amniotic fluid cell population and the main selection for Cd24⁺OB-Cadherin⁺ cells.

### Characterization of kidney derived cell population (methanephric mesenchyme derived cells (MMDC)) from Amniotic Fluid and the four renal subpopulations

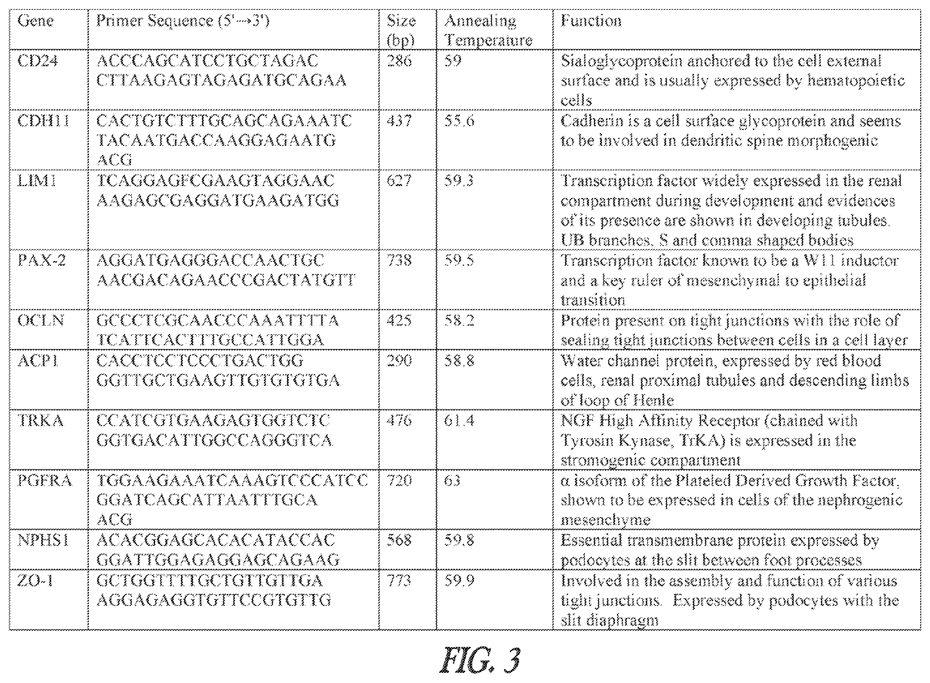

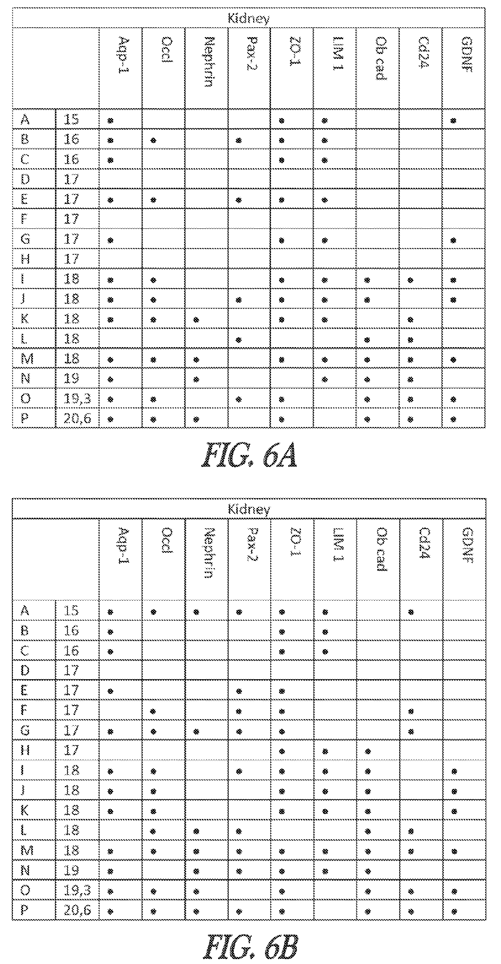

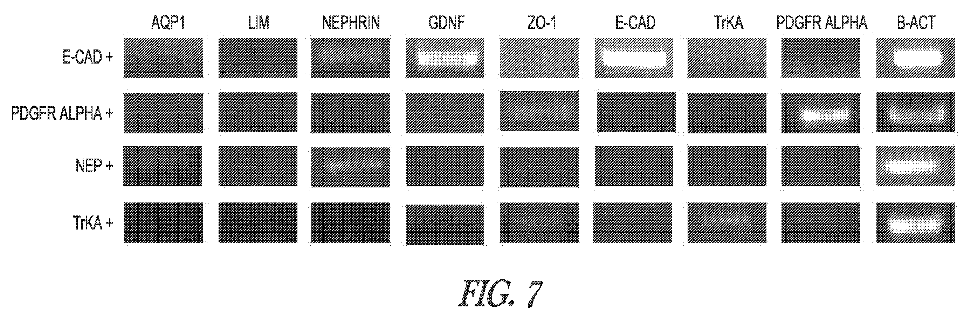

The CD24⁺OB-Cadherin⁺ population (MMDC) were analyzed by RT-PCR and Western blotting analysis for early and mature kidney markers (Figure 3; Figure 6). After the immunoselection, the five subpopulations were analyzed according to the same panel of markers in order to investigate differences and common traits between the Nephrin⁺, TrKA⁺, PDGFR Alpha⁺ and E-Cadherin⁺ MMDC derived populations (Figure 7).

### Analysis and characterization of CD24⁺OB⁻Cadherin⁺ and AKPC by Real Time PCR

The CD24⁺OB⁻Cadherin⁺ population and the five subpopulations were analyzed by Real-Time PCR for *GDNF, WT-1, LIM-1*, *PAX-2*, neprhin, *OCT-4*, *TRKA*, *PDGFRA*, E-cadherin, *ZO-1*, Podocalyxin and occludin.

### Results

### Characterization of amniotic fluid by expression of markers for the three germ layers and progenitor cells

### Human Total Amniotic Fluid Total Cell Population Culture

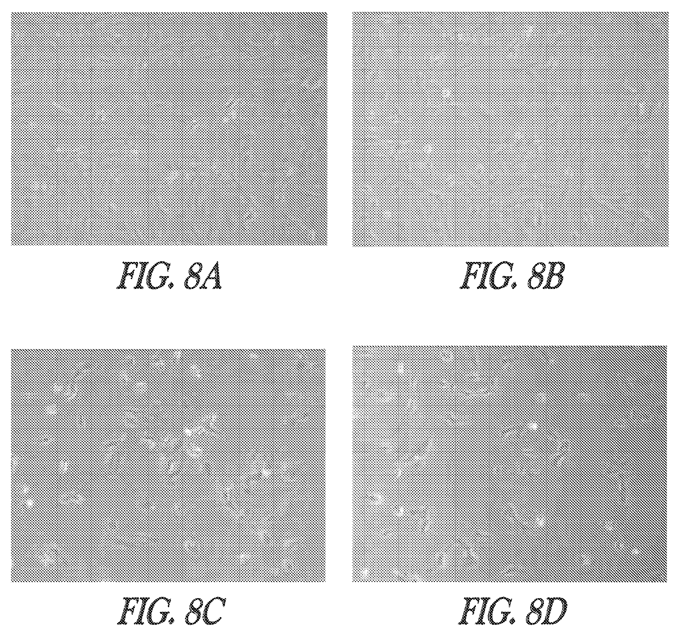

The morphology of the total cell population was heterogeneous with a prevalence of fibroblastoid shapes. The CD24⁺OB-Cadherin⁺ positive selection presents a morphology that differentiates from fibroblast typical shapes (Figure 8A-D). The expansion of the total population of amniotic fluid was possible for up to 10 passages using DMEM, after which the cells ceases to grow. The cells cultured in Amniomax II and Chang media were expanded for more than 50 passages. However, only Chang maintained the original cell morphology. Thus, Chang media was chosen for all experiments.

### Analysis and characterization of human amniotic fluid cells by RT-PCR

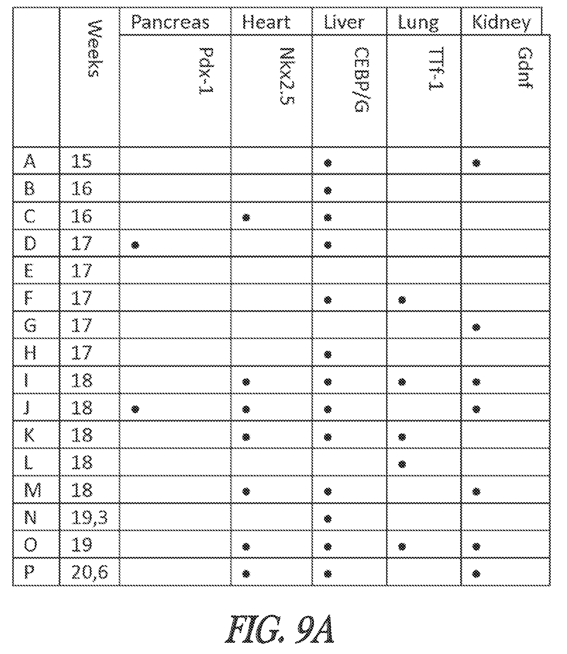

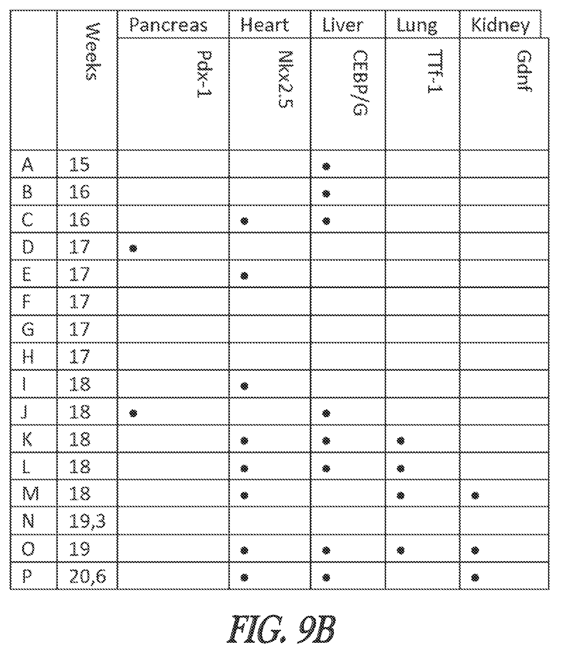

Amniotic Fluid Total Population Cells (AFCP) were categorized by week of gestation (from 15 to 20 weeks) and analyzed using RT-PCR and Western blotting analysis. As shown in Figure 4, the expression of markers for the three germ layers, for pluripotent cells and for mesenchymal and hematopoietic and for early organ progenitor cells of several organs (Figure 9) were analyzed. Expression of genes characteristic of endodermal and mesodermal germ layers was seen to decrease over time while ectodermal markers remained constantly expressed.

Pluripotent markers were expressed in all gestational ages analyzed less than 19 weeks. While the mesenchymal marker *CD90* was expressed in all the time points analyzed, CD34 (marker for haematopoietic lineages) was absent in early samples, but appeared slightly in samples of 18 weeks of gestation or older. Early progenitor markers for different organs were expressed in 18 weeks and in older samples.

### Further quantitative analysis by Real-Time PCR

Four samples at each time point (15-16, 17-18, 19-20 weeks) were analyzed. Some markers, such as Brachyury, *TAL-1*, nephrin and *TRKA* were not expressed in one or more samples Goosecoid and *PDX*-*1* were not found in any sample analyzed.

The epithelial cell marker E-cadherin increased 15-fold at 17-18 weeks of gestation. In contrast, *NCAM* and *FGF5*, while present, did not change significantly over time.

The mesodermal marker Brachyury was expressed at 15-16 weeks in only one sample. *Tal-1* appeared to decrease over time, while *FLK1* increased 4-fold.

The endodermal marker *CXCR-4* increased 3.5 fold between 15-16 and 19-20 weeks while *SOX-17* and *AFP* tended to decrease.

Pluripotency marker *OCT-4* did not change over the range investigated, while *c-kit* increased by 3-fold at 17-18 weeks only to disappear in the older samples.

The hematopoietic marker *CD34* decreased after 17-18 weeks in contrast with the mesenchymal marker *CD90* that increased 2-fold by 17-18 weeks.

Progenitor markers, excluding *PDX-1* which showed no expression, generally increased with age of gestation. *NKX2.5*, an early cardiac marker, increased 6-fold at 19-20 weeks, while the lung/thyroid marker *NKX2.1* doubled its expression at 17-18 weeks and an additional 2.5 fold at 19-20 weeks.

*CEBPG* showed a 5-fold increase in expression at 17-18 weeks compared with the 15-16 and 19-20 weeks of gestation.

### Analysis and characterization of human amniotic fluid cells by Western blotting

The protein expression of the cells showed a decrease in endodermal and mesodermal layers over the course of gestation, while as seen with mRNA expression, ectodermal proteins were constantly present along the gestational age. Pluripotent, hematopoietic and mesenchymal markers followed the same trend seen in the RT-PCR analysis (Figure 4).

The early proteins of progenitor cells from different organs, as shown in Figure 9, showed increased expression in amniotic fluid cells over the course of gestation.

### Selection and characterization of a renal progenitor cells from the whole amniotic fluid

### Analysis and characterization by RT-PCR

Samples expressing GDNF, an early renal marker, were characterized using a panel of antibodies to investigate the expression of markers for early and mature renal differentiation. Presence of kidney derived cells was confirmed by RT-PCR and Western blotting as shown in Figure 6 A-B.

*LIM-1*, Aquaporin-1, *Zo-1* and occluding expression was found in both early and later AF samples. *CD24,* OB-cadherin, *PAX-2*, *GDNF* and neprhin were mostly expressed by 18 weeks of gestation.

### Additional analysis and quantitation by Real-Time PCR

*CD24* and OB-cadherin doubled their expression at 17-18 weeks. Meanwhile *CD24* expression remained unchanged between 17-18 and 19-20 weeks, while over the same period OB-cadherin decreased to the previous level of expression. *Pax-2* increased slightly between 15-16 and 19-20 weeks, while LIM-1 expression did not change. One sample for each time period was positive for nephirn, showing an increase over time, doubling at 19-20 weeks of gestation. *Zo-1* and aquaporin-1 did not change significantly over time, while occludin increased 8-fold at 19-20 weeks.

### Expression of GDNF and PDGFRA was found only in some samples. Immunoseparation of populations and subpopulations from whole amniotic fluid

CD24⁺ OB-Cadherin⁺ selected cells presented a more uniform morphology compared with the total AF cell population. Very long cell processes, typical of podocytes *cultured in vitro* were more apparent in the CD24⁺OB-cadherin⁺ selected cell population. After expansion of the CD24⁺OB-cadherin⁺ population for four passages, subpopulations expressing pdocalyxin, TRKA, neprhin PDGFRA and E-cadherin were obtained by immunoseparation.

### Characterization of Metanephric Mesenchyme derived cells from Amniotic Fluid

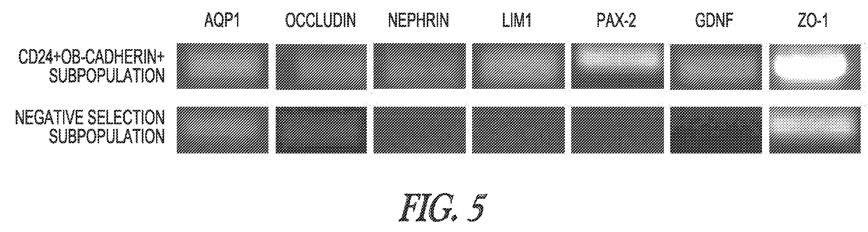

The main population (CD24⁺OB Cadherin⁺) (Figure 5) and the four derived subpopulations were characterized (Figure 7) by RT-PCR and Western blotting for early and mature kidney markers, as well as pluripotent markers, as previously described. Expression of renal markers differed in the investigated populations as shown in the figures. The CD24⁺OB Cadherin⁺E-Cadherin⁺ population expressed E-Cadherin and *GDNF* and was slightly positive for *nephrin.* CD24⁺OB-cadherin⁺nephrin⁺ cells were positive for nephrin (as well asAquaporin 1 and Zona Occludens 1). The population immunoselected for CD24⁺OB-cadherin⁺PDGFR Alpha⁺ was positive for *ZO-1* and *PDGFR Alpha* while the CD24⁺OB-cadherin⁺TrKA⁺ population expressed *TrKA, ZO-1* and a low level of PDGFR Alpha. The CD24⁺OB-cadherin⁺podoclayxin⁺ population was positive for GDNF and slightly for nephrin.

### Analysis and quantitation by Real-Time PCR

The initial population of CD24+OB-cadherin+ and the five subpopulations were analyzed by Real-Time PCR for the expression of specific kidney markers.

The E-cadheri+ subpopulation increased 2-fold in E-cadherin expression, 7-fold in occludin expression and 9-fold in LIM-1 expression. In addition, OCT_4 was increased 12-fold.

PDGFR⁺ subpopulation showed a 2-fold increase in *PDGFRA* expression. TrKA⁺ subpopulation expressed comparable levels of *LIM*-*1*, *PAX-2, OCT-4,* E-cadherin, *PDGFRA* and *TRKA* while Occludin increased about 2-fold. Nephrin⁺ selection showed 11-fold increases in *GDNF*, *LIM*-*1* and *OCT-4*, a 4-fold increase in *PAX-2* and 2-fold increase for Nephrin expression. In addition, a 4-fold increase in Occludin expression and 7-fold increase of *ZO-1. WT-1* was expressed while Podocalyxin expression was not found.

Podocalyxin⁺ population showed a decrease of *LIM-1*, *PAX-2*, *GDNF* and *OCT-4* expression while Podocalyxin expression increased. In addition WT-1 expression was also demonstrated.

### Discussion

Since the discovery of stem cells they have emerged as a promising tool for regenerative medicine. Their characteristics of self renewal and pluripotency have suggested that stem cells may be useful in the repair of injured tissue and the reconstruction of damaged organs, for example, as a useful tool for injured tissue repair and *ex novo* reconstruction of organs. Embryonic Stem Cells (ES cells), for their capability to give rise to the entire cell set of the embryo and to the extraembryonic tissues are widely studied, but ethical issues still preclude their utilization in therapy. Mesenchymal stem cells have been proposed as a useful therapeutic tool, without the ethical and clinical issues that are given by ES cells, becoming a reliable alternative. MSC are pluripotent non-hematopoietic cells able to differentiate into adipocytes, osteocytes, chondrocytes and cardiomyocytes.

Bone marrow and cord blood are well known sources of mesenchymal cells as much as haematopoietic cells, progenitors of cells of the blood stream such as red cells, lymphocytes, macrophages. While haematopoietic cells are used in the therapy for leukaemia and blood disorders, mesenchymal stem cells are undergoing a vast investigation to study their regenerative potential.

Cord blood is presenting some advantages such as the easy access to new samples, the immature state of the cells and the absence of risks in the retrieval of cells. Bone marrow can be a perfect match because the patient could be the donor himself. The autologous cells, after expansion *in vitro,* could be transplanted again in the bone marrow avoiding any immunoreaction.

Adult stem cells have been investigated as possible tools for regeneration of different organs, but the small amount of cells present in the tissues, the difficulty to localize the niches where these cells reside and the limited potentiality in their differentiation have definitely limited their attractiveness.

Amniotic fluid (AF), due to its origin and to the contact with the developing fetus, contains great numbers of various suspended cells, which have been widely used for diagnostic purposes. Recently, stem cells have been isolated from AF that exhibit both embryonic and mesenchymal stem cell characteristics. They are easily attainable, avoid ethical complications, and propagate in culture while maintaining their pluripotential capacity (Prusa and Hengstschlager, 2002). In addition to stem cells, presence of other cell types such as committed progenitor cells and adult cells within amniotic fluid has been postulated.

Stem cells within the AF constitute no more than 1% of the total cell population; little is known about the characterization of other cell types. While cells from the three germ layers attracted the interest of scientists for their capability to give rise to different cell lineages with various and sometimes completely different function, there has not been significant examination of progenitor cells of a committed lineage present in the amniotic fluid. The main focus of this example, therefore, is those cells within AF which have lost their pluripotency and are thus committed to a defined lineage that can give rise to a limited number of different cells.

Samples of human male amniotic fluid were processed with a gestational age ranging between 15 and 20 weeks. Since the composition of amniotic fluid changes over time as the fetus develops during pregnancy, it was determined that, by dividing samples according to gestational age, better information on the cell composition could be obtained.

The development of the embryo includes differentiation of cells through the three germ layers: endoderm, ectoderm and mesoderm. These three pathways in cell differentiation involve loss of potentiality by going from pluripotent into a multipotent state of being. The endodermal layer gives rise to lung, liver, bladder, digestive tract; the ectodermal layer gives rise to brain, spinal cord, skin, hair and eyes; whereas the mesodermal layer gives rise to adipose tissue, bone, skeletal muscle and endothelium.

As demonstrated herein, the inventors were successful in identifying cells expressing all three germ layers, mesenchymal and haematopoietic, progenitors of different organs and in particular, cells presenting specific renal characteristics, including podocyte precursors, epithelial tubular cells and mesangial cells.

Human embryo development follows a precise timetable during gestation. The cells within the AF are committed to various organs at different gestation time points. Within the time range investigated, younger samples express more frequently and at higher levels markers related to mesodermal and endodermal germ layers, while in older samples, expression of these markers decreases in frequency and quantity due to both differentiation of the AF cells over time and the different degree of maturation of the cells detaching from the fetus. In addition, expression of organ-specific markers increases with gestation, presumably due to maturation of organogenesis.

The majority of AF volume derives from fetal urine, and, therefore, it is reasonable to assume that kidney progenitor cells form a major constituent of AF. The expression of renal markers such as *PAX-2*, *LIM*-*1*, nephrin, *PDGFRA, TRKA,* E-cadherin, *CD24* and OB-cadherin showed a clear increase by the end of the 17^{th} week of gestation.

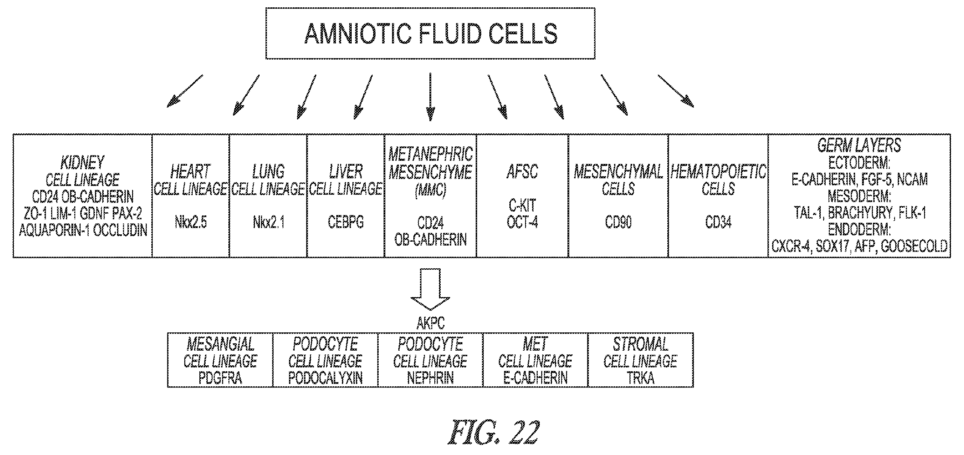

Having shown that kidney progenitors are present among the total cell population of AF, a specific population was selected from AF based on in vivo studies that reported CD24 and OB-cadherin as co-expressed in the developing kidney and in particular the metaneprhic mesenchyme (MM) that, together with the ureteric bud, gives rise to the mature kidney. This population was termed Metanephric Mesenchyme-like Cells (CD24⁺OB-cadherin⁺) as shown in Figure 22. When the MM is induced by the UB, the expression of GDNF1, LIM-1, PAX-2, BMP-2 and other genes become evident, which are expressed also in the CD24⁺OB-cadherin⁺ cell population. Subsequently, with progression of kidney maturation, gene expression varies and begins to be restricted to specific cell lineages. Each cell lineage acquires characteristic traits driven by specific gene expression and indicated by surface markers such as E-cadherin (MET, Mesenchymal-to-Epithelial Transition cells), nephrin (podocytes), podocalyxin (mature podocytes), TRKA (stromogenic cortical mesenchymal cells), and PDGFRA (mesangial cells). These surface markers were therefore used to perform an additional immunoselection from the CD24+OB-cadherin+ population as initially isolated.

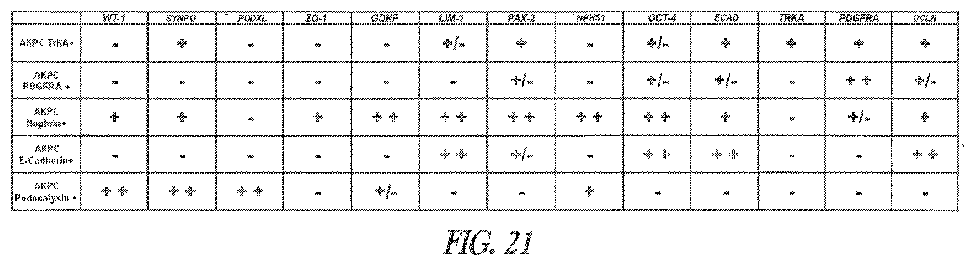

The presence of genes and proteins such as GDNF, WT-1, LIM-1 and PAX-2 in the five CD24+OB-cadherin+ sub-populations determines the fate of renal cells types. Also investigated was the expression of OCT-4 to determine if the sub-selected cells still have multi-differentiation potential. During kidney development, organ specific precursors go through different stages of differentiation in order to reach the mature state. During this maturation process the pluripotent genes are not turned off suddenly, nor are all the maturity specific genes suddenly turned on. In the intermediate state, co-expression of both types of genes occurs. As is shown in summary in Figure 21, the RT-PCR analysis confirmed a specific temporal pattern of renal marker expression for each of the five subpopluations derived rom the CD24+OB-cadherin+ population.

One of the most interesting results is revealed by the expression of WT-1 - expressed *in vivo* in the metanephros in the proximal part of the S-shape body and then exclusively in the mature podocytes. Among all the isolated subpopulations, only the nephric+ and podocalyxin+ cells expressed WT-1, indicating that these are indeed precursor cells for podocytes. In particular, it is important to underscore that genes like PAX-2 or LIM-1 are not expressed in the podocalyxin+ selected cells, but are evident in the nephrin+ cells, suggesting that the neprhin+ cells may represent a more immature podocyte lineage than the podocalyxin-expressing cells. This concept is also supported by the finding that the podocalyxin+ cells do not express OCT-4, while the nephrin+ cells express OCT-4 at high levels. Further, the cultured podocalyxin+ cells showed the typical morphology of cultured human podocytes, presenting primary processes similar to those described by Vogelmann et al. Among the other populations, WT-1 was not expressed, showing no commitment to the podocyte differentiation. In particular, the pattern of expression of the PDGFRA+ cells, with expression of PAX-2 and absence of LIM-1, suggests commitment to the nephrogenic lineage but not current MET. In addition, OCT-4 was highly expressed, a characteristic shared with the TRKA+ population. TRKA+ population was highly positive for PAX-2, LIM-1 and TRKA. In particular, TRKA, expressed solely in stromogenic cortical mesenchymal cells of the developing kidney, proposes TRKA+ cells commitment. E-cadherin+ cells did not express PAX-2, instead, demonstrated expression of LIM-1 and E-cadherin, suggesting their incomplete commitment to the nephrogenic pattern of differentiation.

In summary, in addition to the presence of a small number (1%) of cells with pluripotent characteristics, the composition of the other 99% of AF cells is diverse, with a large subpopulation of cells exhibiting commitment to the defined germ lines or tissue endpoints, ranging from unspecified progenitors to organ specific progenitors as well as mature differentiated cell types. Herein a MM-like population within the AF was identified from which a specific subpopulation could be successfully separated and grown in culture for several passages. The presence and successful identification of specific renal progenitors, in particular podocyte precursors within human AF represents a valuable source of cells for regenerative therapies applicable to a braid range of renal diseases.

### Example 2

### Amniotic Fluid Stem Cells for Kidney Degeneration - Protective Effect of Human Amniotic Fluid Stem Cells in Acute Tubular Necrosis

### Materials and Methods

### Isolation and labelling of c-kit positive stem cells derived from amniotic fluid

Samples of human Amniotic Fluid were obtained from discarded amniocentesis under approval of Childrens Hospital Los Angeles Committee on Clinical Investigations (IRB). No written or verbal consent was required since information obtained about the samples were limited to karyotype and fetus health status. The stem cell population was separated from the general human amniotic cellular milieu using standard Magnetic Sorting (MACS) techniques (Miltenyi Biotech) against cell surface marker c-kit as described by De Coppi et al (2007). Pluripotential characteristics of the clonal and subclonal groups were tested according to protocols also outlined in Atala et al. Clones were then cultured in petri dishes in medium containing *α*-MEM supplemented with 20% Chang B and 2% Chang C solutions, 20% Fetal Bovine Serum, 1% L-Glutamine, and 1% pen-strep antibiotics (GibcoBRL). hAFSC, used for *in vivo* injection, were karyotyped using standard protocols.

Before injections, a clonal hAFSC population was trypsinized in 0.05M trypsin/EDTA solution and centrifuged at 1500rpm for 5 min and then labelled with a cell surface marker CM-Dil (Molecular Probes) following the manufacturer's instructions in order to track the cells during and after injection. Briefly, the cells were incubated with 1mg/ml of CM-Dil for 5 minutes at 37°C followed by an incubation of 15 minutes at 4°C and 3 washes with PBS.

### Acute Tubular Necrosis induction and injection

Rhabdomyolysis-induced ATN (Acute Tubular Necrosis) was induced in female nu/nu mice (Jackson Laboratories, Bar Harbor, ME Harlan) by intramuscular injection with 50% hypertonic glycerol solution (10 ml/kg body wt) (Sigma-Aldrich) following deprivation of water for 22 hours. Controlled intramuscular injection of glycerol was performed under anesthesia by surgically exposing the caudal thigh muscle and slowly injecting the glycerol solution prior to delivery of cells. Animal experiments were performed in adherence to the National Institutes of Health Guide for the Care and Use of Laboratory Animals, with Childrens Hospital Los Angeles Institutional Animal Care and Use Committee approval.

The mice were carefully anesthetized using isofluorane inhalation. Once satisfactory anesthesia was achieved, the mice were prepared for surgery using clorxidine. A small approximate 1cm dorsal incision was made, both kidney's were carefully delivered via the incision, and the hAFSC (1x106 diluted in PBS buffer) carefully injected into the renal cortex of both kidneys with a 30-33 gauge needle using a microinjector Eppendorf TransferMan NK2 Injector (Eppendorf). The kidneys were then replaced into the retroperitoneum, the incision closed with polypropyelne suture (Taper C-1, size 5-0, 90cm), and the mice were allowed to recover from anesthesia. The animal was maintained on a heating pad throughout the period of anesthesia. 0.1 mg/kg of buprenorphine was administered subcutaneously and 1 mg/kg bupivicaine (a local anesthetic) was administered along the incision margins just prior to wound closure to provide post-operative pain relief. The animals were draped to prevent contact of the kidneys with the skin of the animal to reduce risk of peritonitis. Control mice were also injected with PBS.

### Tissue processing

At different time points (from 24 hours to 3 weeks), the injected and the control mice were sacrificed. The kidneys were minced and processed in one of the following ways depending on the analysis performed.

### RNA/DNA extraction

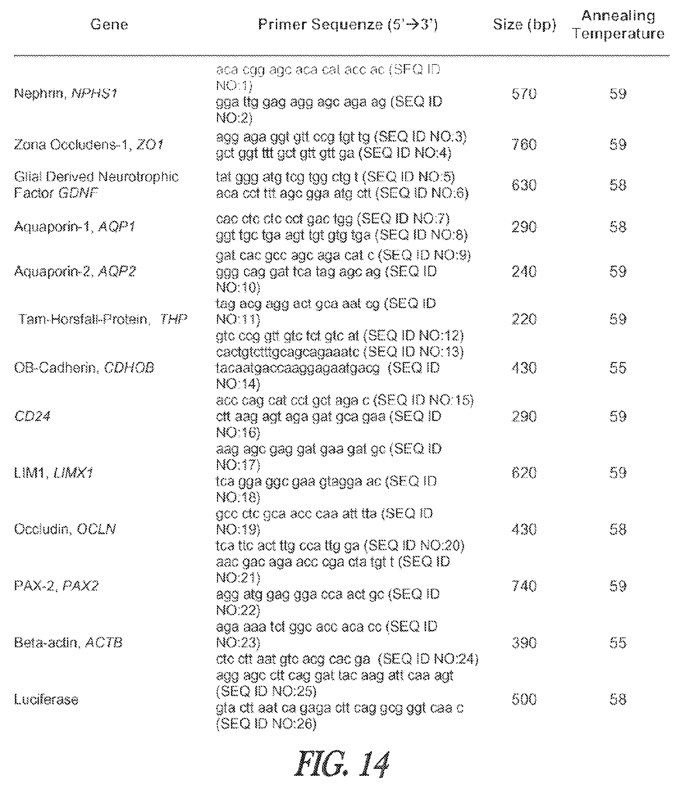

The kidneys were minced in small pieces and the RNA extracted using Qiagen RNeasy kit according to the manufacturer's instructions. Briefly, total mRNA was extracted and reverse transcribed. Amplification of the resulting cDNA was carried out using specific human primers not coding for mouse sequences. A PCR thermal cycler (Eppendorf) was employed after an initial denaturation step at 95°C for 10 min. A denaturation step was performed at 95°C for 30 seconds, followed by an annealing step at the temperature specific for each primer (ranging from 54°C to 60°C) for 45 seconds, and an extension step at 72°C for 45 seconds for a total of 35 cycles. To rule out the possibility of amplifying genomic DNA, RNA samples were treated with a DNA-free kit (Ambion Inc.). Detection of the PCR amplification products was performed by size fractionation on 1% agarose gel electrophoresis. As a housekeeping gene, amplification of fragments of the human β-actin RNA was performed. Primer sequences and predicted sizes of amplicons were as shown in Figure 14.

In order to perform PCR on the genomic DNA to evaluate the presence of the luciferase gene, DNA extraction was performed following standard protocols of the Qiagen DNeasy kit.

### Histology

Kidneys were fixed in 4% buffered paraformaldehyde (Sigma-Aldrich) for 8 hours at 4°C, routinely processed, embedded in paraffin, and sectioned at 5 µm. Briefly, the kidneys were washed in 70% alcohol for 2 hours, followed by two washes in alcohol for 2 hours and placed in toluene, twice for 40 minutes, then one hour in a solution of toluene/paraffin and paraffin overnight. The following morning the kidneys were entirely embedded in paraffin and prepared for sectioning. The sections were stained with hematoxylin and eosin (H&E; Sigma-Aldrich), toluidine blue (Sigma-Aldrich) and Periodic Acid Schiff (PAS; Sigma-Aldrich) following standard histological protocols.

In addition, some kidneys were frozen with liquid nitrogen and stored at -20°C. When necessary they were cryosectioned at 5 µm and then used for immuno-histochemistry.

### Labeling of the AFSC with luciferase-bioluminescent detection

hAFSC were transduced with a lentiviral vector (SMPU-R-MNCU3-LUC based on HIV-1 that transduces the firefly luciferase gene) made by the Vector Facility at Childrens Hospital Los Angeles following standard protocols. Two cycles of transduction were performed by removing old medium and adding new virus supernatant and medium. Twenty-four hours after the initial transduction, cells were thoroughly washed 3 times with phosphate-buffered saline (PBS) before transplantation or *in vitro* analysis. Before *in vivo* injections, a *simple in vitro* test was employed to determine the minimum amount of hAFSC detectable by bioluminescence. Different concentrations of the cells ranging from 5x10⁴ to 2x10⁶ were evaluated. In addition, it was confirmed that after 20 passages in culture the cells were still expressing the luciferase gene by PCR. 10-week old *nu*/*nu* mice obtained from Jackson Laboratories were injected directly into the kidney with luciferase-transduced hAFSC (1 x 10⁶ cells/mouse diluted in PBS). *In vivo* optical imaging was performed with a prototype IVIS 3-dimensional bioluminescence/fluorescence optical imaging system (Xenogen, Alameda, CA) at different time points.

Prior to imaging, each mouse was given an intravenous injection of luciferin (Promega) at a dose of 125 mg/kg, as described (Wang et al., 2003). General anesthesia was then induced with 5% isoflurane and the mouse was placed in the light-tight heated chamber; anesthesia was continued during the procedure with 2% isoflurane introduced via a nose cone. The imaging system consisted of a cooled, back-thinned charge-coupled device (CCD) camera to capture both a visible light photograph of the animal taken with light-emitting diodes and a luminescent image. A rotating mirror and translatable animal stage allowed for images to be acquired over 360°.

### Immunostaining

Frozen and paraffin slides were stained with fluorescence. Paraffin slides were deparaffinized, placed in 1% Triton for 5 min (if the antigen was nuclear) and briefly washed in PBS. The paraffin slides were then placed in Vector Antigen Retrieval Solution (Vector Laboratories) for three cycles. The frozen slides were fixed for 5 min in 80% methanol. After Avidin/Biotin blocking (Vector Laboratories) a second block was carried for 30 min using the appropriate 5% normal serum in PBS. The slides were incubated in primary antibody (Dolichos biflorus and Peanut agglutinin from Vector Laboratories, Luciferase from Promega and Glial Derived Neutrophic Factor and Aquaporin 2 from Santa Cruz) solution for 1 hr at room temperature or overnight at 4°C. The slides were then washed in PBS for 5 min x 3. Secondary antibody (Vector Laboratories) concentration was 1:200 in 5% normal serum. Slides were incubated in this solution for 1.5 hours at room temperature, followed by 5 min x 3 PBS. The appropriate fluorescent marker (Texas Red or Fluorescein Avidin DCS from Vector Laboratories) was then applied in a concentration of 1:500 in PBS buffer for 5-10 min, followed by a final 5 min x 3 PBS wash. TUNEL staining (Roche, Applied-Science) was performed to determine the presence of apoptotic cells. Briefly, the cells were incubated at 37°C for one hour with the TUNEL reagent and then washed in PBS. Slides were mounted with Vector DAPI mounting medium (Vector Laboratories). In the experimental groups, the number of positive apoptotic nuclei was counted per 300 nuclei and hAFSC treated animals were compared to untreated controls. Values are mean ± SD. A Leica DM RA fluorescent microscope was used in conjunction with Open Lab 3.1.5 software to image the staining.

### Blood Collection, Creatinine and BUN measurements

The facial vein was lanced with a 5mm point length animal lancet and blood was collected using standard protocols approved by the Animal Core Facility at Childrens Hospital of Los Angeles and Saban Research Institute. Animals were divided into different groups as follows: 1. Ten animals for measuring baseline creatinine levels; 2. Ten animals that underwent ATN (Acute Tubular Necrosis) with no injection of hAFSC; 3. Ten animals that underwent ATN and intrarenal injection of hAFSC after 2 hours of glycerol injection; 4. Ten animals that underwent induction of ATN with glycerol and intrareanl injection of PBS after 2 hours of glycerol injection.

The blood samples (30*µ*L) were collected into plasma separation tubes with lithium heparin. They were centrifuged at 13,000-RPM for 3 min and the plasma (upper layer) was removed and stored at -80°C until analysis. A maximum of 15% of circulating blood was sampled in a given 14-day period (total blood volume ∼0.6% of total body weight). Post-damage measurements were obtained every 24 hours. The blood samples were used to monitor renal function, by analyzing creatinine levels and BUN levels. ELISA was performed according to the manufacture for both creatinine (BioAssay Systems Cat # DUCT-500) and BUN (BioAssay System Cat # DIUR-500) using 30*µ*L serum samples loaded into 96-well microplates. Comparison between groups were made using an unpaired t test. A value of p< 0.05 was considered statistically significant. Analyses were done using GraphPad Prism software. Data are shown as mean ± SD.

### Morphological studies.

Kidney sections were prepared at 4 µm thickness by a routine procedure and stained with PAS reagents as described above. The kidney sections were divided into six main groups: 1. Mice that underwent ATN with no injection of hAFSC sacrificed at 24 hours; 2. Mice that underwent ATN and injection of hAFSC after 2 hours of glycerol injection sacrificed at 24 hours; 3. Mice that underwent ATN with no injection of hAFSC sacrificed at 48 hours 4. Mice that underwent ATN and injection of hAFSC after 2 hours of glycerol injection sacrificed at 48 hours 5. Mice that underwent ATN with no injection of hAFSC sacrificed at 72 hours; and 6. Mice that underwent ATN and injection of hAFSC after 2 hours of glycerol injection sacrificed at 72 hours.

Tubular injury was evaluated based on three major parameters using PAS staining: 1. Disruption of the tubular membrane; 2. Disruption of brush borders; and 3. Cast formation. In the experimental groups, the damaged tubules were counted as a fraction of the total number of tubules present in the section using consecutive, non-overlapping fields of PAS-stained specimens. The percentage of damaged tubules was estimated without knowledge of the experimental group. Values are mean ± SD.

### Cytokine Analysis

To examine pro-inflammatory and anti-inflammatory molecules that were generated after glycerol-induced ATN (with or without injection of cells), human and mouse cytokine levels were measured in digested mouse kidneys using a multiple cytokines array technique (Proteome Profiler Array Kit). Briefly, kidney tissue was homogenized in a cell lysis buffer, and the homogenates were centrifuged at 12,000 rpm for 15 min at 4°C. Total protein concentration in each supernatant was determined using a Cytokine Array Kit (for human cat. no. ARY005 and for mouse cat. no. ARY006), as suggested by the protocol (R&D Systems, Inc.). The data were analyzed using the Array Vision Program (R&D Systems, Inc).

### Results

### hAFSC phenotype and karyotype before injection

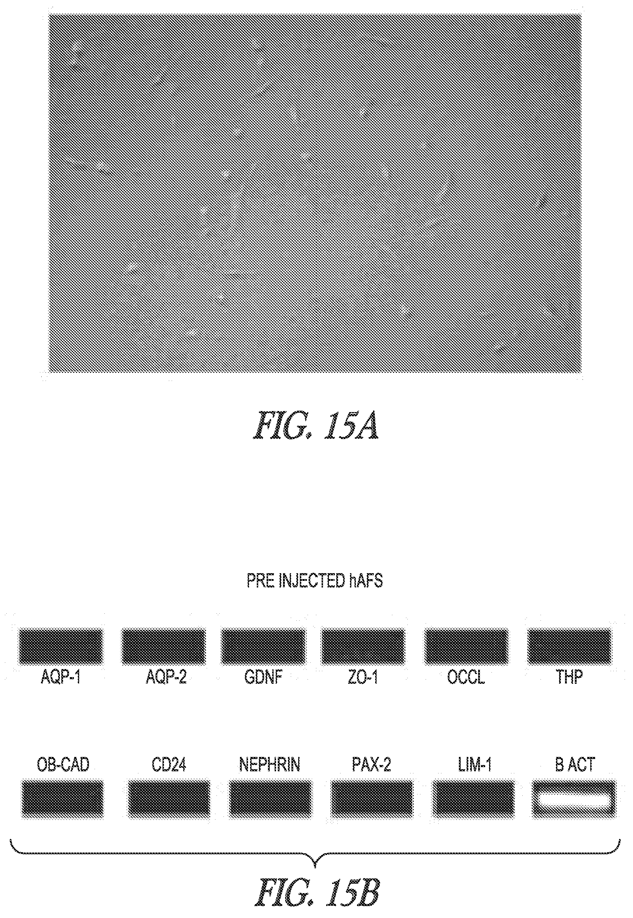

hAFSCs before injection present a fibroblastoid shape as shown in Fig. 15A. hAFSC were analyzed for the expression of early and late kidney markers before injection. As shown in Fig 15B, hAFSC were negative for many kidney markers, ranging from transcription factors expressed during early kidney development to late differentiation markers. This allowed the confirmation that the hAFSC are not specifically committed to kidney progenitor cells when cultured *in vitro.*

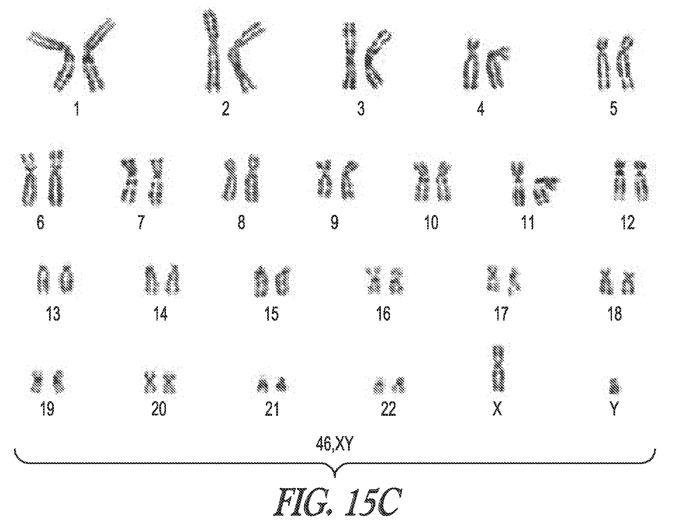

The cells were tested to confirm a normal karyotype before *in vivo* application in order to exclude chromosomal abnormalities that could compromise their pluripotential capability (Fig. 15C).

### Glycerol induced muscle damge damage and Acute Tubular Necrosis (ATN) Hematoxylin and Eosin (H&E), Period Acid Schiff staining (PAS), TUNEL

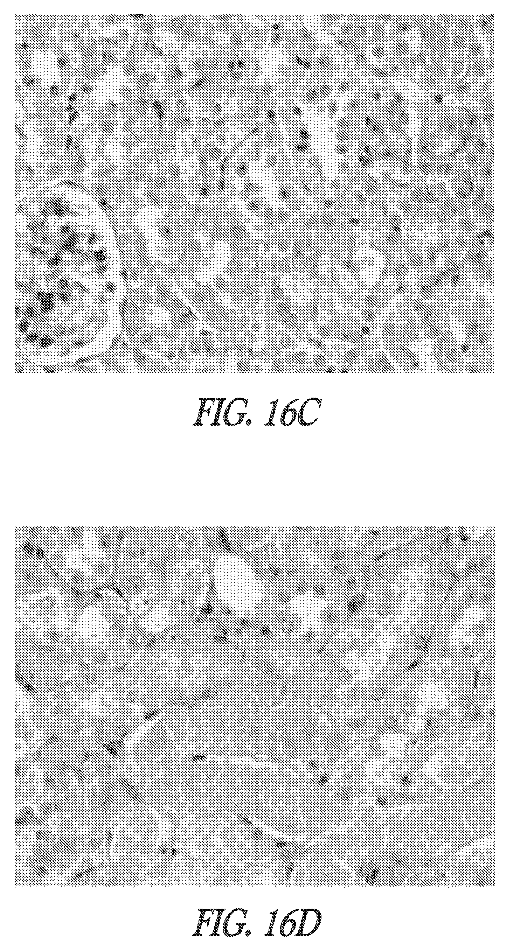

Fig 16A demonstrates the normal morphology of a mouse kidney (nu/nu) before any damage. The distinction between the medulla and the cortex is clearly evident, and the tubules are intact as well as the glomeruli. Fig. 16B shows morphology of the kidney 3 days after the intramuscular injection of glycerol. ATN caused a marked disorganization in the structure so that medulla and cortex were not distinguishable. The normal structure of proximal and distal tubules were destroyed (with cast formation), while most of the glomeruli remain intact. This type of damage is typical of the injury induced by rhabdomyolysis, where the main structures of the kidneys that undergo failure are the tubules and not the glomeruli. The main pathophysiologic mechanisms in this well established model are renal vasoconstriction, intraluminar cast formation and the direct heme-protein induced cytotoxicity with the production of free radicals that enhances ischemic damage (FIGs 16C and 16D).

There was an increase in apoptotic cells (TUNEL staining) when compared with the control (that did not undergo glycerol induced muscle damage). Additionally, the difference in the number of apoptotic cells present in the treated glycerol mice when compared with the untreated control mice was highly significant.

### In vivo detection of hAFSC by bioluminescence

Before *in vivo* injection, hAFSC were transduced with a lentivirus coding for luciferase with stable expression of the transgene over many population doublings. Fig. 17A shows cells not infected, as a control, versus infected cells exposed to luciferin (the luciferase substrate) to confirm the presence of the signal under bioluminescence detection after 20 population doublings. In Fig. 17B an *in vitro* experiment determined that the smallest number of cells optically detected under bioluminescence was 1x10⁵ cells.

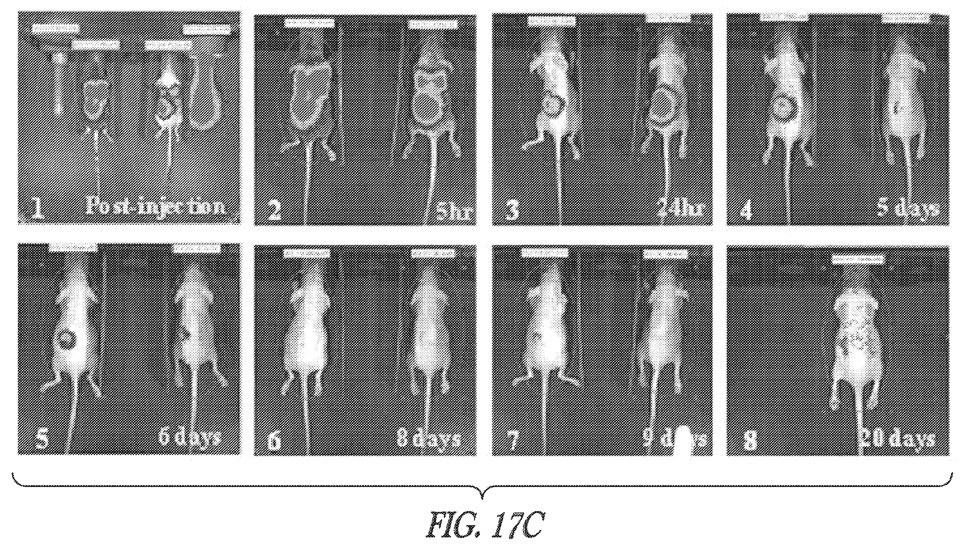

1.2 x10⁶ hAFSC were injected directly into the right kidney after damage induction (Fig. 17C) were easily detected. The left kidney was used as a control. The signal was clearly evident and spread into multiple zones of the body (panel 1-2), such s the lung over the first few days. The signal for hAFSC in the area of the kidney could be seen at 24 hours after injection (panel 3). The kidney signal was strongest at 48 hours and 72 hours, and persisted for up to 6 days (panel 4-5), after which the signal began to diminish over the next several days (panel 6-7). However, 21 days after injection, the signal was evident again in the area of the kidney (panel 8).

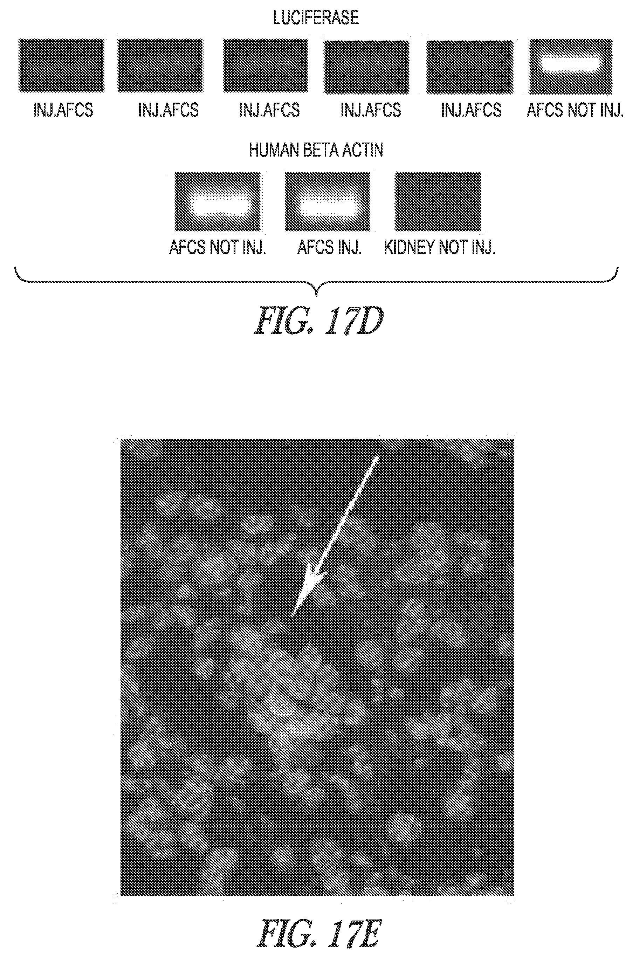

DNA extraction and PCR were performed at 21 days on injected and non-injected kidneys in order to determine the presence of luciferase. Results show that luciferase and human *ATCB* DNA was present only in the injected kidney tissue (Fig. 17D), as confirmed by the absence of the housekeeping *A TCB.* DNA was extracted from the entire injected kidney. The results were also confirmed with a positive immunostaining against luciferase as shown in Fig. 17E.

### Detection of hAFSC in damaged kidneys by immunohistochemistry and gene expression

The presence of injected hAFSC was evaluated histologically. Frozen sections at 1 week after injection confirmed the presence of hAFSC detected by red fluorescence of the surface marker CM-Dil (Fig. 18A). Several instances where the CM-Dil signal from the hAFSC overlapped with the fluorescent-staining of a kidney marker were observed as follows: luciferase positive hAFSC were double stained for Aquaporin2, Peanut Agglutinin, Dolichus Biflorus Agglutinin at 3 weeks after injection, indicating that hAFSC are able to differentiate into cells expressing adult proximal and distal tubular agglutinins (Fig. 18B-D). In a few cases hAFSC were also found in glomerular structures expressing (Glial Derived Neurotrophic Factor (GDNF; Fig. 18E), indicating that the stem cells were also able to express early glomerular markers of differentiation.

After 21 days, RT-PCR was performed using human specific primers on the harvested kidneys and expression of several specific human kidney genes (early and late markers of differentiation) by the hAFSC in the injected kidneys were identified: Nephrin, Aquaporin 2, Pax-2, and Occl, when compared to hAFSC before injection (Fig. 18F).

### Creatinine and Blood Urea Nitrogen (BUN) measurements

A control group of 10 *nu*/*nu* mice was used to determine the basal level of serum creatinine before any treatment, which averaged 0.6 mg/dl. After intramuscular injection glycerol on day 0, creatinine levels increased to as high as 1.10 mg/dl, showing a peak between 48 and 72 hours after injection.

Similarly, the level of BUN (basal level of 27mg/dl) increased after glycerol injection and the peak was detected around 48 and 72 hours. The concentration of both creatinine and BUN returned to normal level after 3 weeks. Further analysis showed no statistical in significant difference in creatinine and BUN levels between the animals that were injected with saline vehicle solution versus no injection following intramuscular glycerol, and therefore these groups were pooled for statistical analysis. n contrast, animals subjected to damage induced with intramuscular glycerol and receiving an intrarenal hAFSC injection demonstrated no increase in levels of creatinine or BUN during the expected acute phase of injury.

### Morphological studies

An increase in the number of damaged tubules is seen from 24 hours to 72 hours in the glycerol-treated animals. By 72 hours the damage is more severe due to the cast formation within damaged tubules. In glycerol-injected animals treated with hAFSC the number of damaged tubules increased at 48 hours compared to the animals that were not injected with stem cells, but by 72 hours the number of damaged tubules decreased significantly. Two-factor ANOVA showed a highly significant effect of time (p= 0.03) and interaction of time with treatment (p=0.01).

### Immuno-cytokine profile

Since the salutary effect of hAFSC injection occurred during the acute phase of ATN, the protective effect may involve acute changes in the kidney's cytokine milieu. The cytokine profile of human as well as mouse cytokines expressed in the kidney at 24 hours and 48 hours after intramuscular injection of glycerol is compared to undamaged controls and mice with glycerol-injected and intrarenal injection of hAFSC. Cytokine levels are shown as a comprehensive suite of sequential bar graphs (Mean and SD) in Figure 20. For relative ease of interpretation, the different cytokines were displayed as four broad functional clusters based on their principal immunological functions: 1. Anti-inflammatory; 2. Pro-inflammatory; 3. Chemoattractants; and 4. Multiple biological effects. For each individual cytokine, the bars in the figure, from left to right, show: control cytokine levels; ATN kidney at 24 hours; and ATN kidney plus hAFSC injection at 24 hours (shown as the sum of mouse derived plus hAFSC derived human cytokine levels). This order of display is then repeated at 48 hours.

The mouse specific cytokine assay does not cross react with the human cytokine assay. This was confirmed by incubating mouse digested kidneys with membranes specific for human cytokines and conversely incubating hAFSC with membranes specific for mouse cytokines (data not shown).

The mouse tissue is exposed to the activity of both human and mouse cytokines at both time points shown (24 hours and 48 hours). When viewed in this fashion it becomes clear that the major trend of the analyzed combined cytokine levels is a significant increase in the early cytokine response. Thus, 24 and 48 hours following intramuscular injection of glycerol, at the time of peaking kidney damage, mice without hAFSC injection had significant elevation (as much as 5 or 6-fold) of cytokine expression level across all 4 classes of cytokine. However, when glycerol-treated mice were injected with hAFSC, an even greater elevation of cytokine levels was evident, particularly at 24 hours when both mouse and human cytokine levels were combined. However, by 48 hours, this trend in combined cytokine levels is reversed so that the majority of combined cytokine levels are either reduced significantly or no longer elevated relative to the kidneys that did not receive hAFSC. Further, by 48 hours the relative contribution of human versus mouse cytokines is also reversed, with the human component of the cytokine milieu being relatively small.

### Abbreviations

hAFSC: human Amniotic Fluid Stem Cells; ATN: Acute Tubular Necrosis; H&E: Hematoxylin and Eosin; PAS: Periodic Acid Schiff; BUN: Blood Urea Nitrogen; AQP1: Aquaporin 1; AQP2: Aquaporin 2; GDNF: Glial Derived Neutrofic Factor; ZO-1: Zona Occludens-1; OCLN: Occuldin; THP: Tamm- Horsfall-Protein; CDHOB: OB-Cadherin; ACTB: *β*-actin.

### Discussion

Acute Tubular Necrosis (ATN) causes severe damage to the epithelial tubular cells of kidney that can lead to End Stage Renal Disease (ESRD). A protective role for hAFSC injected directly into kidneys with glycerol-induced ATN was demonstrated herein. In recent years studies have supported a potential role of stem cells, mainly mesenchymal stem cells derived from bone marrow or of kidney-specific progenitors (Lin, 2007; Al-Awqati and Oliver, 2006), to ameliorate renal injury. Transplanted bone marrow stem cells were found integrated into damaged kidneys (Gupta et al., 2002; Poulsom et al., 2001). Morigi et al. (2004, 2006) and Herrara et al. (2004) demonstrated that meshenchymal stem cells (MSC) are capable of integrating into damaged tubules and speculated that MSC derived from bone marrow have the ability to differentiate into renal epithelial cells. However, whether there was any physiologic benefit of incorporation of these cells within damaged tubules of the kidney is unclear. In contrast, other groups have shown that MSC have a role in restoring function to damaged kidneys through mechanisms other than incorporation and replication (Duffield et al., 2005; Lin et al., 2005). Bonventre et al. (2008) underscored the importance of MSC in renal repair and raised the possibility that MSC may mediate repair by affecting the inflammatory process following acute renal injury.

A new stem population derived from amniotic fluid has been characterized (De Coppi et al., 2007). These c-kit⁺ cells can differentiate into cells derived from all three germ *layers in vitro* and showed potential for similar *in vivo* differentiation. hAFSC have the potential to integrate into embryonic kidneys and participate in key steps of nephrogenesis, indicating that hAFSC can be induced to a renal fate when placed in an appropriate environment (Perin et al., 2007).

In the present study for acute renal failure (*nu*/*nu* mice) the amount of glycerol required for induction of ATN was 50% higher than the dose needed in a wild type mouse (data not shown). This suggests that T-deficient mice (nude) may be protected as compared to wild type mice against glycerol-rhabdomyolysis-induced ATN (Burne et al., 2001). Glycerol induced ATN involves a complex sequence of events where myoglobin, released from the damaged muscle, damages the epithelial cells of the proximal tubules, causing cast formation, vasoconstriction and decreased glomerular filtration pressure. The high level of apoptotic cells, the increase levels of creatine and BUN, and the histological analysis confirmed the presence of ATN in the instant model.

The number of hAFSC that survived after injection is reduced over time as evidenced by the luciferase detection. Nevertheless, injected hAFSC can differentiate into tubule epithelial cells. hAFSC were found within the damaged kidneys three weeks post injection located within damaged tubules, and expressing epithelial markers, as measured by both immunohistochemistry and RT-PCR using specific human antibodies and human primers. These markers were not present in hAFSC *in vitro* prior to injection.

It was further demonstrated that injected hAFSC are also capable of expressing kidney genes such as PAX2 and NPHS1, indicating that they can be induced to commit toward renal differentiation. Furthermore, in some instances, injected hAFSC cells could express Glial Derived Neurotrophic Factor (GDNF), which is expressed during very early kidney development; GDNF is not usually expressed in the adult kidney.

Whether hAFSC can modulate kidney function after damage was also evaluated, as reflected in the serum creatinine and BUN. When hAFSC were injected during the established acute phase of the damage (between 48-72 hours after the injection of the glycerol), the levels of creatinine and BUN did not decrease (data not shown). This implies that injection of hAFSC when damage is already established is too late to attenuate the damage. In contrast, when hAFSC were injected into the kidney on the same day of glycerol injection no peaks in creatinine and BUN levels were observed, underscoring the potential protective effects of hAFSC. Thus, hAFSC can, when injected early enough, in this case contemporaneously with the time of injury, attenuate acute renal injury.

Furthermore, the histology analysis demonstrated that by 72 hours after glycerol injection, the kidneys that were injected with the hAFSC show fewer damaged tubules compared with the glycerol-injected kidneys not treated with hAFSC. There was less disruption of tubular membranes and no cast formation in the hAFSC-treated animals. Thus, it appears that injection of hAFSC accelerates the proliferation of epithelial tubular cells that were partially damaged and prevents additional apoptosis. This mechanism of protection lead to an overall better maintenance of the tubular structure, thus avoiding the increase in BUN and creatinine typically seen in glycerol-induced ATN.

During acute renal injury the immune-response plays a role especially in the first 48 hours; damaged kidney endothelial cells attract leukocytes, vasomediators are released with injury, and epithelial cells of the tubule produce proinflammatory and chemotactic cytokines (Bonventre et al. 2003). Bonventre et al. (2008) and Lin et al. (2007) have speculated recently that the mechanism by which bone marrow stem cells contribute to renal repair was by attenuating the immune response, rather than through integration or differentiation of the stem cells into the cells of the damaged organ. Togel et al. (2005) have shown that injection of MSC is protective against ischemic renal injury as early as 24 hours, based on measurement of serum creatinine levels. They also speculated that the protection in these animals was not through integration and differentiation of the injected MSC, because of the very short period of time with which a protective response was observed.

Herein it was demonstrated that the beneficial effect of hAFSC injection occurred early in the course of ATN. Therefore, to further investigate the potential mechanisms by which hAFSC enhance renal protection, intrarenal cytokines were examined in order to determine whether there is a general change in inflammatory cytokine pattern in mice that were treated with hAFSC compared to untreated mice during the first 48 hours, thus when the immune-system acts in a very significant manner in determining the course of the acute damage.

In the animals that were subjected only to glycerol injection a significant increase in kidney cytokine expression was demonstrated when compared with control mice that were not treated. This demonstrates that in acute ATN, the kidney responds with a brisk outpouring of cytokines. When mice were injected with hAFSC, an even greater elevation of cytokine levels was evident, particularly at the earlier (24 hours) time point, when compared with the cytokines levels measured in kidney treated only with glycerol. Thus, a function of hAFSC may be to augment the kidney cytokine milieu early in the course of ATN.

Moreover by 48 hours, this trend in combined cytokine levels is reversed so that the majority of combined cytokine levels are either reduced significantly or no longer elevated relative to the kidneys that did not receive hAFSC. Further, by 48 hours the relative contribution of human versus mouse cytokines is also reversed, with the human component of the cytokine milieu being relatively small.

With a few exceptions, most human cytokines are also active on mouse cells (Maliszewski et al. 1998; Hu-Li et al. 1987; Liu et al. 1995; Schwabe et al. 1996; Kennedy et al. 1996; De Haan et al. 2000), so both the human and mouse cytokines likely affect the kidney milieu. This may be relevant since it is likely that the complex interaction of cytokines derived from both the injected human cells as well as the endogenous mouse cytokines may play a role in any protective effects.

Therefore, hAFSC, when injected directly into the kidney in a mouse model of ATN, can be recruited as previously shown in two mouse models of lung injury (Carraro et al. 2008). Thus, hAFSC can home to injury sites, where they protect damaged tissue from further injury and accelerate repair through cytokine-mediated paracrine mechanisms. It is believed that the cytokines secreted by hAFSC work in synergy with the endogenous mouse cytokines to promote and maintain overall homeostasis of the tissues and thus interact with the inflammatory environment favoring damage resolution, thus allowing the prevention of progression of the acute phase in glycerol-induced ATN.

In addition, at 48 hours after injection of hAFSC into the ATN kidney, the major contribution to the pattern of cytokine expression is from mouse cytokines, when compared with the combined cytokine levels at 24 hours, at which point the relative contribution of human cytokines is very high. This suggests that the human cytokines play a role in the earliest phase of renal response to injury.

In conclusion, a trophic effect of hAFSC on resident kidney cells that survive a toxic injury was demonstrated, rather than via direct repopulation of the damaged structures, even if they show potential of differentiation into epithelial tubular cells over time. Early direct injection of hAFSC into the kidney strongly ameliorates ATN injury as reflected by more rapid resolution of tubular structural damage and by normalized creatinine and BUN levels. In addition, the data show evidence of immunomodulatory effects of hAFSC at a very early time point, comparable in magnitude to endogenous cytokine production. Thus, hAFSC have application for therapeutic purposes in kidney diseases, including a pluripotential cell source for tissue regeneration and a viable alternative for whole organ engineering.

### BIBLIOGRAPHY

Al-Awqati Q, Oliver JA. Stem Cell Rev. 2006;2(3):181-184.
Bates C.M. Pediatr Nephrol (2007) 22:343-349.
Barisoni and Mundel (2003) Am J Nephrol 23:353-60.
Bonventre JV. Semin Nephrol. Sep 2003;23(5):439-448.
Bossolasco P, et al. Cell Res. 2006 Apr;16(4):329-36.
Bruce SJ, et al. Differentiation. 2007 Jun;75(5):337-49. Epub 2007 Feb 5.
Burne MJ et al. J Clin Invest. Nov 2001;108(9):1283-1290.
Carraro et al. Stem Cells 2008 Nov;26(11):2902-11.
Cellini C, Xu J, Buchmiller TL. J Pediatr Gastroenterol Nutr. 2006 Sep;43(3):291-8.
Challen GA et al. J Am Soc Nephrol. 2004 Sep;15(9):2344-57.
Challen GA et al. (2006) J Am Soc Nephrol 17:1896-912.
Chamberlain G, Fox J, Ashton B, Middleton J. Concise Review: Mesenchymal Stem Cells: Their Phenotype, Differentiation Capacity, Immunological Features, and Potential for Homing Stem Cells. 2007 Nov;25(11):2739-49.
De Coppi P et al. Nat Biotechnol. 2007 Jan;25(1):100-6.
De Coppi P. et al. Nat Biotechnol. Jan 2007;25(1):100-106.
De Hann G et al. Br J Haematol. 2000 Sep;110(3):638-46.
Duffield JS, Bonventre JV. Kidney Int. Nov 2005;68(5):1956-1961.
Duffield JS et al. J Clin Invest. Jul 2005;115(7):1743-1755.
Fogo AB. Pediatr Nephrol. 2007 Dec;22(12):2011-22. Epub 2007 Jul 24.
Gosden CM., Br Med Bull. 1983 Oct;39(4):348-54
Gupta S et al. Kidney Int. Oct 2002;62(4):1285-1290.
Heart Development. Edited by Richard P. Harvey and Nadia Rosenthal. 530 pp., illustrated. San Diego, Calif., Academic Press, 1999.
Herrera MB et al. Int J Mol Med. Dec 2004; 14(6):1035-1041.
Hoehn H, Salk D. Methods Cell Biol. 1982;26:11-34.
Horster MF et al. (1999) Physiol Rev 79:1157-91.
Hu-Li J et al. J Exp Med. 1987 Jan 1;165(1):157-72.
Humphreys BD, Bonventre JV. Annu Rev Med. Feb 18 2008;59:311-325.
Kanwar YS, et al. Exp Biol Med (Maywood). 2008 Jan;233(1):4-11.
Kashtan CE. 1998 Sep;9(9):1736-50.
Kennedy J et al. J Interferon Cytokine Res. 1996 Aug;16(8):611-7.
Krtil J, et al. Kidney Blood Press Res. 2007;30(3):162-74.
Lanza, R. P., et al. Nat Biotechnol, 20: 689,2002.
Lin F, Moran A, Igarashi P. J Clin Invest. Jul 2005;115(7):1756-1764.
Lin F. Pediatr Nephrol. Nov 9 2007.
Liu C et al. J Interferon Cytokine Res. 1995 Nov;15(11):985-92.
Maliszewski CR et al. Mol Immunol. 1988 Sep;25(9):843-50.
McLaughlin D, et al. J Neurosci Res. 2006 May 15;83(7):1190-200.
Morigi et al. Stem Cells. 2008;26(8):2075-82.
Morigi M et al. Cell Transplant. 2006;15 Suppl 1:S111-117.
Morigi M et al. J Am Soc Nephrol. Jul 2004; 15(7):1794-1804.
National Kidney and Urologic Diseases Information Clearinghouse. Kidney and Urologic Diseases Statistics for the United States, In National Institute of Health, National Institute of Diabetes and Digestive and Kidney Diseases website: http://kidney.niddk.nih.gov/kudiseases/pubs/kustats/index.htm#kpClearinghouse.
Nyengaard JR, Bendtsen Anat Rec 232 : 194-201, 1992.
Oliver and Al Awqati (2000) Kidney Int 57:2167-8.
Pavenstadt H et al. (2003) Physiol Rev 83:252-307.
Perin L et al. Cell Prolif. Dec 2007;40(6):936-948.
Piper K, et al. Journal of Endocrinology, 2004 Apr;181(1):11-23.
Post M, Copland I. Acta Pharmacol Sin 2002 Oct; 23 Supplement: 4-7.
Poulsom R et al. J Pathol. 2001 Sep;195(2):229-35.
Prusa AR, Hengstschlager M. Med Sci Monit. 2002 Nov;8(11):RA253-7.
Quaggin SE, Kreidberg JA. Development. 2008 Feb;135(4):609-20. Epub 2008 Jan 9.
Quondamatteo F, et al. Histochem Cell Biol (1997) 107:223-228
Rubinstein P.Why cord blood?Hum Immunol. 2006 Jun;67(6):398-404. Epub 2006 Mar 30.
Schwabe M et al. Cell Immunol. 1996 Fed 25;168(1):117-21.
Schrier RW, et al. J Clin Invest. Jul 2004;114(1):5-14.
Semedo P et al. Int Immunopharmacol. 2009 June;9(6):677-82.
Stocum D.L., 2001. Wound Rep. Reg. 9: 429-442.
Streubel B, et al. Wien Med Wochenschr. 1996;146(9-10):216-7.
Takano K, et al. J Exp Med. 2007 May;212(1):81-90.
Tarnowski M, Sieron A Med Sci Monit, 2006; 12(8): RA154-163.
Thadhani R et al. N Engl J Med. May 30 1996;334(22):1448-1460.
Thomson J.A. et al., 1998. Science 282: 1145-1147.
Togel F et al. Am J Physiol Renal Physiol. Jul 2005;289(1):F31-42.
Torricelli F, et al. Ital J Anat Embryol. 1993 Apr-Jun;98(2): 119-26. Erratum in: Arch Ital Anat Embriol 1993 Jul-Sep;98(3):215.
Tsangaris G et al. Electrophoresis 2004, 25, 1168-1173
Underwood M et al. J of Perinatology (2005) 25, 341-348.
US Renal Data System. USRDS 2002. Annual Data Report : Atlas of End.-Stage Renal Desease in the United States. Bethesda., MD : National Institute of Health, National Institute of Diabetes and Digestive and Kidney Diseases.
Vainio S, Lin Y. Nature Reviews Genetics 3, 533-543 (July 2002).
Wang X et al. Blood. Nov 15 2003;102(10):3478-3482.
Warburton et al. Proc Am Thorac 2008;5:703-6.
Weiss ML, Troyer DL. Stem cells in the umbilical cord. The Midwest Institute for Comparative Stem Cell Biology and the Department of Anatomy and Physiology, Kansas State University College of Veterinary Medicine, Manhattan, KS 66506-5602, USA. weiss@vet.ksu.edu.
Vogelmann SU et al. (2003) Am J Physiol Renal Physiol 285(1):F40-8.
Vogetseder A, et al. Am J Physiol Cell Physiol. Oct 3 2007.
Yokoo T et al. J Am Soc Nephrol. Apr 2006;17(4):1026-1034.

All publications, patents and patent applications are incorporated herein by reference. While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. Amniotic fluid derived renal progenitor cells positive for CD24, OB-cadherin and podocalixin, being an isolated and purified population or a clonal population.

2. The cells of claim 1, which can be induced to differentiate *ex vivo.*

3. The cells of any preceding claim, which can be induced to differentiate *in vivo.*

4. The cells of any preceding claim, which can be induced to differentiate to metanephric, podocyte, stromogenic mesenchymal or mesanglial cells.

5. The cells of any preceding claim for use in treating kidney disease or injury.

6. The cells of claims 1-4 for the use of claim 5 in treating kidney disease in a mammal.

7. The cells of claims 1-4 for the use of claim 5 in treating kidney disease in a human.

8. A composition comprising amniotic fluid derived renal progenitor cells as claimed in claims 1-4 and a pharmaceutically acceptable carrier and/or culture medium.

9. A method of producing a population of amniotic fluid derived renal progenitor cells comprising selecting CD24, OB-cadherin and podocalixin positive cells from an amniotic fluid sample.

10. The method of claim 9, wherein the selecting is performed using an antibody against CD24, an antibody against OB-cadherin, and an antibody against podocalixin.

11. The method of claim 9 or 10, wherein the selecting is by fluorescence activated cell sorting or high gradient magnetic selection.

12. The method of any of claims 9 to 11, further comprising any of the following:
(i) proliferating a population of cells enriched for amniotic fluid derived renal progenitor cells comprising introducing at least one selected cell to a culture medium and proliferating said at least one selected cell in the culture medium;
(ii) differentiating the isolated and purified population of amniotic fluid derived renal progenitor cells positive for CD24, OB-cadherin and podocalixin by contacting said population with at least one differentiation factor;
(iii) storing the isolated and purified population of amniotic fluid derived renal progenitor cells by cryopreservation.

## Patentansprüche

1. Aus Amnionflüssigkeit gewonnene, renale Vorläuferzellen, die für CD24, OB-Cadherin und Podocalixin positiv und eine isolierte und aufgereinigte Population oder eine klonale Population sind.

2. Zellen nach Anspruch 1, die induziert werden können, um ex *vivo* zu differenzieren.

3. Zellen nach einem der vorhergehenden Ansprüche, die induziert werden können, um *in vivo* zu differenzieren.

4. Zellen nach einem der vorhergehenden Ansprüche, die induziert werden können, um zu metanephrischen Zellen, Podocytenzellen, mesenchymalen Stroma-Zellen oder Mesangialzellen zu differenzieren.

5. Zellen nach einem der vorhergehenden Ansprüche zur Verwendung bei der Behandlung einer Nierenerkrankung oder -verletzung.

6. Zellen nach den Ansprüchen 1-4 zur Verwendung nach Anspruch 5 bei der Behandlung einer Nierenerkrankung in einem Säugetier.

7. Zellen nach den Ansprüchen 1-4 zur Verwendung nach Anspruch 5 bei der Behandlung einer Nierenerkrankung in einem Menschen.

8. Zusammensetzung, die aus Amnionflüssigkeit gewonnene, renale Vorläuferzellen nach den Ansprüchen 1-4 und einen pharmazeutisch akzeptablen Träger und/oder Kulturmedium umfasst.

9. Verfahren zum Herstellen einer Population von aus Amnionflüssigkeit gewonnenen, renalen Vorläuferzellen, umfassend das Auswählen aus einer Amnionflüssigkeitsprobe von Zellen, die für CD24, OB-Cadherin und Podocalixin positiv sind.

10. Verfahren nach Anspruch 9, wobei das Auswählen unter Verwendung eines Antikörpers gegen CD24, eines Antikörpers gegen OB-Cadherin und eines Antikörpers gegen Podocalixin durchgeführt wird.

11. Verfahren nach Anspruch 9 oder 10, wobei das Auswählen durch fluoreszenzaktivierte Zellsortierung oder Hochgradienten-Magnet-Selektion erfolgt.

12. Verfahren nach einem der Ansprüche 9 bis 11, das ferner irgendetwas von dem Folgenden umfasst:
(i) Vermehren von einer Zellpopulation, die mit aus Amnionflüssigkeit gewonnenen, renalen Vorläuferzellen angereichert ist, umfassend das Einführen von mindestens einer ausgewählten Zelle in ein Kulturmedium und das Vermehren der mindestens einen Zelle in dem Kulturmedium;
(ii) Differenzieren der isolierten und aufgereinigten Population von aus Amnionflüssigkeit gewonnenen, renalen Vorläuferzellen, die für CD24, OB-Cadherin und Podocalixin positiv sind, durch das In-Kontakt-Bringen der Population mit mindestens einem Differenzierungsfaktor;
(iii) Aufbewahren der isolierten und aufgereinigten Population von aus Amnionflüssigkeit gewonnenen, renalen Vorläuferzellen durch Kryokonservierung.

## Revendications

1. Cellules progénitrices rénales dérivées de liquide amniotique positives pour CD24, OB-cadhérine et podocalixine, qui sont une population isolée et purifiée ou une population clonale.

2. Cellules selon la revendication 1, qui peuvent être induites pour se différencier ex *vivo.*

3. Cellules selon l'une quelconque des revendications précédentes, qui peuvent être induites pour se différencier *in vivo.*

4. Cellules selon l'une quelconque des revendications précédentes, qui peuvent être induites pour se différencier en cellules métanéphriques, podocytes, mésenchymateuses stromogéniques ou mésangliales.

5. Cellules selon l'une quelconque des revendications précédentes, pour une utilisation dans le traitement d'une maladie ou d'une lésion du rein.

6. Cellules selon les revendications 1 à 4, pour l'utilisation selon la revendication 5 dans le traitement d'une maladie du rein chez un mammifère.

7. Cellules selon les revendications 1 à 4 pour l'utilisation selon la revendication 5, dans le traitement d'une maladie du rein chez un être humain.

8. Composition comprenant des cellules progénitrices rénales dérivées de liquide amniotique selon les revendications 1 à 4, et un support et/ou un milieu de culture pharmaceutiquement acceptable(s).

9. Procédé de production d'une population de cellules progénitrices rénales dérivées de liquide amniotique comprenant la sélection de cellules positives pour CD24, OB-cadhérine et podocalixine à partir d'un échantillon de liquide amniotique.

10. Procédé selon la revendication 9, dans lequel la sélection est réalisée à l'aide d'un anticorps dirigé contre CD24, d'un anticorps dirigé contre OB-cadhérine, et d'un anticorps dirigé contre la podocalixine.

11. Procédé selon la revendication 9 ou 10, dans lequel la sélection se fait par tri de cellules activées par fluorescence ou par sélection sous gradient magnétique élevé.

12. Procédé selon l'une quelconque des revendications 9 à 11, comprenant en outre l'un quelconque de ce qui suit :
(i) la prolifération d'une population de cellules enrichies en cellules progénitrices rénales dérivées de liquide amniotique comprenant l'introduction d'au moins une cellule choisie dans un milieu de culture et la prolifération de ladite au moins une cellule choisie dans le milieu de culture ;
(ii) la différenciation de la population isolée et purifiée de cellules progénitrices rénales dérivées de liquide amniotique positives pour CD24, OB-cadhérine et podocalixine par mise en contact de ladite population avec au moins un facteur de différenciation ;
(iii) le stockage de la population isolée et purifiée de cellules progénitrices rénales dérivées de liquide amniotique par cryoconservation.
